# EUROPEAN PATENT APPLICATION

(11) **EP 2 987 489 A1**
(43) Date of publication of application: **24.02.2016**
(21) Application number: 14785332.9
(22) Date of filing: 18.04.2014
(51) Int. Cl.: A61K 31/4439, A61K 9/30, A61K 9/52, A61P 25/28

(54) **CONTROLLED-RELEASE DRUG FORMULATION**

(30) Priority: 19.04.2013 JP 2013088940
(71) Applicant: Takeda Pharmaceutical Company Limited, Osaka-shi, Osaka 541-0045 (JP)
(72) Inventor: MURAKAWA, Yusuke, Osaka-shi Osaka 532-0024 (JP); TSUTSUMI, Shunichiro, Osaka-shi Osaka 532-0024 (JP); TAKENAKA, Kaoru, Osaka-shi Osaka 532-0024 (JP); INOO, Kanako, Osaka-shi Osaka 532-0024 (JP)
(74) Representative: Casalonga
(86) International application number: PCT/JP2014/061090
(87) International publication number: WO 2014/171542

(57) **Abstract**

The present invention provides a controlled-release solid preparation containing pioglitazone or a salt thereof as an active ingredient, and having superior sustainability.

A controlled-release solid preparation containing pioglitazone or a salt thereof as an active ingredient, which is composed of an immediate-release part and a sustained-release part in combination.

## Description

### Technical Field

The present invention relates to a controlled-release solid preparation containing pioglitazone or a salt thereof.

### (Background of the Invention)

Patent document 1 discloses a composition for controlled release where the speed of release of an active ingredient is controlled at 2 or more different phases, which comprises release control section A comprising a proton pump inhibitor as the active ingredient, which can control the speed of release of the active ingredient to a specified level, and release control section B comprising a proton pump inhibitor as the active ingredient, which can control the speed of release of the active ingredient to a specified level lower than the release speed in the release control section A, wherein the release of the active ingredient in the release control section B is earlier than the release of the active ingredient in the release control section A.

Patent document 2 discloses a gastric floating type drug slow release solid preparation constituted of (A) a volume swelling preparation part containing a water-swellable gel forming polymer and a water-expandable foaming agent component dispersed in the polymer, and (B) a drug-containing preparation part, which is laminated on the preparation part (A) or embedded in the preparation part (A) such that a part thereof is exposed, and further coated with a water-permeable and elastic film composed of a water-insoluble polymer.

However, the both documents do not describe a controlled-release preparation containing pioglitazone or a salt thereof as an active ingredient.

### [Document List]

### [patent documents]

patent document 1: JP-A-2004-300149
patent document 2: JP-A-6-24959

### SUMMARY OF THE INVENTION

### Problems to be Solved by the Invention

Pioglitazone hydrochloride has been widely used in the medical field as an antidiabetic drug extremely superior in both the therapeutic effect and safety for many years, and received high evaluation. Specifically, it is provided as an immediate-release preparation in the medical field.

Application of pioglitazone hydrochloride to delay the onset of Alzheimer's disease has been considered. The present inventors decided to study a controlled release type preparation in consideration of a long-term dosing, including prophylactic use for Alzheimer's disease, of pioglitazone hydrochloride to patients.

Controlled-release preparation is desired to show the following effects.
(1) It is a preparation capable of sustained release of a drug, which can provide stable efficacy by a sustained release of the drug even when the dose thereof is low.
(2) It can control the maximum drug concentration (hereinafter sometimes to be described as Cmax) (e.g., can suppress to a lower level than immediate-release preparation).
(3) In addition, it can achieve an area under the blood drug concentration-time curve (hereinafter sometimes to be described as AUC) comparable to that of an immediate-release preparation, which in turn affords sufficient efficacy.
(4) It is also expected to provide a preparation capable of standing physical stimulation of diet (not easily influenced by diet).

The present invention aims to provide a controlled-release preparation containing pioglitazone or a salt thereof, which provides the effects of the above-mentioned (1) - (4). Means of Solving the Problems

The present inventors have conducted various studies of a preparation in an attempt to achieve the object and found, in the consideration process of solution preparations and sustained-release preparations, that a preparation having the following properties is preferable, also based on the dissolution property and pharmacokinetic test results (Cmax, AUC and the like) of dog and human.

### (preparation having preferable property)

When kinetics of pioglitazone in the blood after oral administration to pentagastrin-treated beagle is measured under fasting conditions and the area under the blood drug concentration-time curve (AUC₀₋₂₄ₕᵣ) is calculated by the trapezoidal rule based on the measurement results of plasma concentration before administration, and 0.5, 1, 2, 4, 6, 8, 12 hr and 24 hr thereafter, a preparation having Cmax of 20 - 140 ng/mL and AUC₀₋₂₄ₕᵣ of 100 - 510 ng/mL, more preferably a preparation having Cmax of 40 - 130 ng/mL and AUC₀₋₂₄ₕᵣ of 160 - 480 ng/mL, further preferably a preparation having Cmax of 60 - 125 ng/mL and AUC₀₋₂₄ₕᵣ of 220 - 450 ng/mL, on administration of 1 mg of pioglitazone or 1 mg based on pioglitazone.

In the present specification, "administration of 1 mg of pioglitazone or 1 mg based on pioglitazone" means values of Cmax and AUC₀₋₂₄ₕᵣ as they are when a controlled-release solid preparation containing 1 mg of pioglitazone is administered, and values obtained by proportion of the determined values of Cmax and AUC₀₋₂₄ₕᵣ into those of 1 mg administration when a controlled-release solid preparation containing other dose is administered (e.g., when a preparation containing 4 mg of pioglitazone is administered, one-fourth of the determined values).

The present inventors have conducted various studies of formulating in an attempt to solve a new problem of creation of a preparation having such preferable preparation characteristics and completed the present invention.

Accordingly, the present invention provides the following.
[1] A controlled-release solid preparation comprising pioglitazone or a salt thereof in an amount of 0.1 - 8 mg as pioglitazone per preparation unit, which is composed of an immediate-release part, and a sustained-release part in combination.
[2] The controlled-release solid preparation of the above-mentioned [1], wherein pioglitazone or a salt thereof is contained in one or each of the immediate-release part and the sustained-release part.
[3] The controlled-release solid preparation of the above-mentioned [2], having a structure wherein the immediate-release part and the sustained-release part are laminated.
[4] The controlled-release solid preparation of the above-mentioned [3], wherein pioglitazone or a salt thereof is contained in each of the immediate-release part and the sustained-release part.
[5] The controlled-release solid preparation of the above-mentioned [4], wherein a weight ratio of pioglitazone or a salt thereof contained in the immediate-release part and pioglitazone or a salt thereof contained in the sustained-release part is 1:0.1 - 1:10 in the amount of pioglitazone.
[6] The controlled-release solid preparation of the above-mentioned [4], wherein a weight ratio of pioglitazone or a salt thereof contained in the immediate-release part and pioglitazone or a salt thereof contained in the sustained-release part is 1:0.5 - 1:2 in the amount of pioglitazone.
[7] The controlled-release solid preparation of any of the above-mentioned [3] - [6], comprising a gel forming polymer in the sustained-release part.
[8] The controlled-release solid preparation of any of the above-mentioned [4] - [7], having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.
[9] The controlled-release solid preparation of the above-mentioned [2], having a structure wherein one of the immediate-release part and the sustained-release part constitutes an inner core and the other constitutes an outer layer covering the inner core.
[10] The controlled-release solid preparation of the above-mentioned [9], wherein the inner core has a tablet structure.
[11] The controlled-release solid preparation of the above-mentioned [10], wherein pioglitazone or a salt thereof is contained in each of the immediate-release part and the sustained-release part.
[12] The controlled-release solid preparation of the above-mentioned [11], wherein a weight ratio of pioglitazone or a salt thereof contained in the immediate-release part and pioglitazone or a salt thereof contained in the sustained-release part is 1:0.1 - 1:10 in the amount of pioglitazone.
[13] The controlled-release solid preparation of the above-mentioned [11], wherein a weight ratio of pioglitazone or a salt thereof contained in the immediate-release part and pioglitazone or a salt thereof contained in the sustained-release part is 1:0.5 - 1:2 in the amount of pioglitazone.
[14] The controlled-release solid preparation of any of the above-mentioned [10] - [13], comprising a gel forming polymer in the sustained-release part.
[15] The controlled-release solid preparation of any of the above-mentioned [10] - [14], having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.
[16] The controlled-release solid preparation of the above-mentioned [9], wherein the inner core has a tablet structure and the outer layer has a membrane structure.
[17] The controlled-release solid preparation of the above-mentioned [16], wherein the inner core is the immediate-release part and the outer layer is a sustained-release coating layer comprising a water-soluble polymer and an insoluble polymer.
[18] The controlled-release solid preparation of the above-mentioned [17], wherein pioglitazone or a salt thereof is contained only in the immediate-release part.
[19] The controlled-release solid preparation of any of the above-mentioned [16] - [18], having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.
[20] The controlled-release solid preparation of any of the above-mentioned [3] - [19], which is a tablet.
[21] The controlled-release solid preparation of the above-mentioned [2], wherein one or each of the immediate-release part and the sustained-release part has a granule structure.
[22] The controlled-release solid preparation of the above-mentioned [21], wherein the each of the immediate-release part and the sustained-release part has a granule structure.
[23] The controlled-release solid preparation of the above-mentioned [22], wherein the sustained-release part is a sustained-release granule obtained by coating an immediate-release granule with a coating layer containing a polymer providing sustained release property.
[24] The controlled-release solid preparation of the above-mentioned [22], wherein the sustained-release part is a sustained-release granule obtained by coating an immediate-release granule with a coating layer containing an enteric polymer.
[25] The controlled-release solid preparation of the above-mentioned [24], wherein the coating layer containing an enteric polymer is dissolved at pH 5 - 7.
[26] The controlled-release solid preparation of the above-mentioned [24], wherein the coating layer containing an enteric polymer is dissolved at pH 5.5 - 6.5.
[27] The controlled-release solid preparation of any of the above-mentioned [22] - [26], wherein pioglitazone or a salt thereof is contained in each of the immediate-release part and the sustained-release part.
[28] The controlled-release solid preparation of the above-mentioned [27], wherein a weight ratio of pioglitazone or a salt thereof contained in the immediate-release part and pioglitazone or a salt thereof contained in the sustained-release part is 1:0.1 - 1:3 in the amount of pioglitazone.
[29] The controlled-release solid preparation of the above-mentioned [27], wherein a weight ratio of pioglitazone or a salt thereof contained in the immediate-release part and pioglitazone or a salt thereof contained in the sustained-release part is 1:0.5 - 1:1 in the amount of pioglitazone.
[30] The controlled-release solid preparation of any of the above-mentioned [22] and [24] - [29], having a dissolution rate of pioglitazone of 10-90% on average at 2 hr time point and 30-110% on average at 4 hr time point, preferably 20-80% on average at 2 hr time point and 40-110% on average at 4 hr time point, in a dissolution test comprising dropping one capsule obtained by filling granules constituting the immediate-release part and granules constituting the sustained-release part, in 900 mL of a hydrochloric acid solution (pH 1.2), evaluating same by the USP basket method at rotation number of 100 rpm, 37°C and, 2 hr after dropping the capsule, changing the test solution to 900 mL of a phosphate buffer (pH 6.8)/0.1% CTAB solution and evaluating same under similar conditions.
[31] The controlled-release solid preparation of the above-mentioned [21], having a granule structure wherein the immediate-release part has a granule structure, and the sustained-release part constitutes an outer layer covering the granule structure.
[32] The controlled-release solid preparation of the above-mentioned [31], having a granule structure wherein the immediate-release part has a granule structure, and the sustained-release part is applied thereon as a coating layer.
[33] The controlled-release solid preparation of the above-mentioned [32], wherein the sustained-release part is a coating layer containing a polymer providing sustained release property.
[34] The controlled-release solid preparation of the above-mentioned [33], wherein the polymer providing sustained release property is a combination of a water-soluble polymer and an insoluble polymer.
[35] The controlled-release solid preparation of the above-mentioned [32], wherein the sustained-release part is a coating layer containing an enteric polymer.
[36] The controlled-release solid preparation of any of the above-mentioned [31] - [35], wherein pioglitazone or a salt thereof is contained only in the immediate-release part.
[37] The controlled-release solid preparation of any of the above-mentioned [31] - [34] and [36], having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP basket method at rotation number of 100 rpm, 37°C using a 0.3M potassium chloride buffer (pH 2) as a test solution.
[38] The controlled-release solid preparation of any of the above-mentioned [21] - [37], which is a capsule filled with the immediate-release part and the sustained-release part.
[39] The controlled-release solid preparation of the above-mentioned [21], which is a tablet comprising the immediate-release part and the sustained-release part dispersed therein.
[40] The controlled-release solid preparation of the above-mentioned [21], which has a structure wherein the sustained-release part has a granule structure, the immediate-release part constitutes an outer layer covering same, and the sustained-release part is dispersed in the outer layer.
[41] A controlled-release solid preparation, having a structure wherein an inner core has a layer structure wherein a highly swellable polymer layer and one or plural immediate-release layers containing pioglitazone or a salt thereof in an amount of 0.1 - 8 mg as pioglitazone per preparation unit are laminated, the inner core is coated with a semipermeable membrane as an outer layer, and the semipermeable membrane on the drug-containing immediate-release layer side of the inner core has a minute hole for drug release.
[42] The controlled-release solid preparation of the above-mentioned [41], which is a tablet.
[43] A controlled-release solid preparation comprising a water-swellable gel forming polymer, a water-expandable foaming agent component, and pioglitazone or a salt thereof in an amount of 0.1 - 8 mg as pioglitazone per preparation unit.
[44] The controlled-release solid preparation of the above-mentioned [43], which has a matrix structure wherein the water-swellable gel forming polymer, the water-expandable foaming agent component, and pioglitazone or a salt thereof are dispersed.
[45] The controlled-release solid preparation of the above-mentioned [43], comprising a volume swelling preparation part (A) containing the water-expandable foaming agent component, and a preparation part (B) containing the water-swellable gel forming polymer and pioglitazone or a salt thereof, which is laminated on the preparation part (A) or embedded in the preparation part (A) such that a part thereof is exposed.
[46] The controlled-release solid preparation of any of the above-mentioned [43] - [45], having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at rotation number of 50 rpm, 37°C using a 0.3M potassium chloride buffer (pH 2) as a test solution.
[47] The controlled-release solid preparation of any of the above-mentioned [1] - [46], comprising pioglitazone or a salt thereof in an amount of 0.5 - 8 mg as pioglitazone per preparation unit.
[48] The controlled-release solid preparation of any of the above-mentioned [1] - [46], comprising pioglitazone or a salt thereof in an amount of 0.5 - 6 mg as pioglitazone per preparation unit.
[49] The controlled-release solid preparation of any of the above-mentioned [1] - [46], comprising pioglitazone or a salt thereof in an amount of 0.5 - 1 mg as pioglitazone per preparation unit.
[50] The controlled-release solid preparation of any of the above-mentioned [1] - [49], which has Cmax of 20 - 140 ng/mL and the area under the blood drug concentration-time curve (AUC₀₋₂₄ₕᵣ) of 100 - 510 ng/mL on administration of 1 mg of pioglitazone or 1 mg based on pioglitazone, when pioglitazone is orally administered to a pentagastrin-treated beagle under fasting conditions and kinetics of pioglitazone in blood is measured after the administration, and AUC₀₋₂₄ₕᵣ is calculated by the trapezoidal rule based on the measurement results of plasma concentration before administration, and after 0.5, 1, 2, 4, 6, 8, 12 hr and 24 hr.
[51] A method for the prophylaxis and/or treatment of Alzheimer's dementia, comprising administering the controlled-release solid preparation of any of the above-mentioned [1] - [50] to a subject in need of the administration.
   The present invention also relates to the following.
[52] A controlled-release solid preparation comprising pioglitazone or a salt thereof as an active ingredient, which is composed of an immediate-release part, and a sustained-release part in combination.
[53] The controlled-release solid preparation of the above-mentioned [52], wherein pioglitazone or a salt thereof is contained in one or each of the immediate-release part and the sustained-release part.
[54] The controlled-release solid preparation of the above-mentioned [53], having a structure wherein the immediate-release part and the sustained-release part are laminated.
[55] The controlled-release solid preparation of the above-mentioned [53], having a structure wherein one of the immediate-release part and the sustained-release part constitutes an inner core, and the other constitutes an outer layer covering the inner core.
[56] The controlled-release solid preparation of the above-mentioned [55], wherein the inner core has a tablet structure.
[57] The controlled-release solid preparation of the above-mentioned [55], having a structure wherein the inner core has a layer structure wherein a highly swellable polymer layer and one or plural immediate-release layers containing pioglitazone or a salt thereof are laminated, the outer layer part of the inner core is coated with a semipermeable membrane as an outer layer, and the semipermeable membrane on the drug-containing immediate-release layer side of the inner core has a minute hole for drug release.
[58] The controlled-release solid preparation of any of the above-mentioned [55] - [57], wherein the outer layer has a membrane structure.
[59] The controlled-release solid preparation of the above-mentioned [58], wherein the outer layer is a coating layer comprising a water-soluble polymer and an insoluble polymer.
[60] The controlled-release solid preparation of any of the above-mentioned [52] - [59], which is a tablet.
[61] The controlled-release solid preparation of the above-mentioned [53], wherein one or each of the immediate-release part and the sustained-release part has a granule structure.
[62] The controlled-release solid preparation of the above-mentioned [61], wherein the each of the immediate-release part and the sustained-release part has a granule structure.
[63] The controlled-release solid preparation of the above-mentioned [62], which is a capsule filled with the immediate-release part and the sustained-release part.
[64] The controlled-release solid preparation of the above-mentioned [61] or [62], which is a tablet comprising the immediate-release part and the sustained-release part dispersed therein.
[65] The controlled-release solid preparation of the above-mentioned [61], which has a structure wherein the sustained-release part has a granule structure, the immediate-release part constitutes an outer layer covering same, and the sustained-release part is dispersed in the outer layer.
[66] The controlled-release solid preparation of the above-mentioned [65], which is a tablet.
[67] The controlled-release solid preparation of any of the above-mentioned [61] - [66], wherein the sustained-release part is a sustained-release granule obtained by coating an immediate-release granule with a coating layer containing a polymer providing sustained release property.
[68] The controlled-release solid preparation of any of the above-mentioned [61] - [66], wherein the sustained-release part is a sustained-release granule obtained by coating an immediate-release granule with a coating layer containing an enteric polymer.
[69] A controlled-release solid preparation having a structure wherein an inner core has a layer structure wherein a highly swellable polymer layer and one or plural immediate-release layers containing pioglitazone or a salt thereof are laminated, an outer layer part of the inner core is coated with a semipermeable membrane as an outer layer, and the semipermeable membrane on the drug-containing immediate-release layer side of the inner core has a minute hole for drug release.
[70] The controlled-release solid preparation of the above-mentioned [69], which is a tablet.
[71] A controlled-release solid preparation comprising a volume swelling preparation part (A) containing a water-swellable gel forming polymer, and a preparation part (B) containing pioglitazone or a salt thereof, which is laminated on the preparation part (A) or embedded in the preparation part (A) such that a part thereof is exposed.
[72] The controlled-release solid preparation of the above-mentioned [71], wherein the volume swelling preparation part (A) further comprises a water-expandable foaming agent component dispersed in the water-swellable gel forming polymer.
[73] The controlled-release solid preparation of any of the above-mentioned [52] - [72], comprising pioglitazone or a salt thereof in an amount of 0.5 - 8 mg as pioglitazone per preparation unit.
[74] The controlled-release solid preparation of any of the above-mentioned [52] - [73], which has Cmax of 20 - 140 ng/mL and the area under the blood drug concentration-time curve (AUC₀₋₂₄ₕᵣ) of 100 - 510 ng/mL, more preferably Cmax of 40 - 130 ng/mL and AUC₀₋₂₄ₕᵣ of 160 - 480 ng/mL, further preferably Cmax of 60 - 125 ng/mL and AUC₀₋₂₄ₕᵣ of 220 - 450 ng/mL, when pioglitazone is orally administered to a pentagastrin-treated beagle under fasting conditions and kinetics of pioglitazone in blood is measured after the administration, and AUC₀₋₂₄ₕᵣ is calculated by the trapezoidal rule based on the measurement results of plasma concentration before administration, and after 0.5, 1, 2, 4, 6, 8, 12 hr and 24 hr.

### Effect of the Invention

The controlled-release solid preparation of the present invention has the following effects. (1) It is a preparation capable of sustained release of a drug, which is expected to afford stable efficacy by a sustained release of the drug even when the dose thereof is low. (2) It can control Cmax (e.g., can suppress to a lower level than immediate-release preparation). (3) It can achieve AUC comparable to that of an immediate-release preparation. (4) It is also expected to provide a preparation capable of standing physical stimulation by diet (not easily influenced by diet).

### Brief Description of the Drawings

Fig. 1 shows the results of Experimental Example 1.
Fig. 2-1 shows the results of Experimental Example 2.
Fig. 2-2 shows the results of Experimental Example 2.
Fig. 3 shows the results of Experimental Example 3.
Fig. 4 shows the results of Experimental Example 4.
Fig. 5 shows the results of Experimental Example 5.
Fig. 6 shows the results of Experimental Example 6.
Fig. 7 shows the results of Experimental Example 7.
Fig. 8-1 shows the results of Experimental Example 8.
Fig. 8-2 shows the results of Experimental Example 8.
Fig. 9 shows the results of Experimental Example 9.
Fig. 10 shows the results of Experimental Example 10.

### (Detailed Description of the Invention)

The present invention is explained in detail in the following.

In the "pioglitazone or a salt thereof" to be used in the controlled-release solid preparation of the present invention, examples of the salt of pioglitazone include pharmacologically acceptable salts, for example, salts with inorganic acids, salts with organic acids, salts with acidic amino acids and the like.

Preferable examples of the salts with inorganic acids include salts with hydrochloric acid, hydrobromic acid, nitric acid, sulfuric acid, phosphoric acid and the like.

Preferable examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salts with acidic amino acids include salts with aspartic acid, glutamic acid and the like.

Pioglitazone may be any of anhydride and hydrate, and may be further labeled with isotope (e.g., ³H, ¹⁴C, ³⁵S, ¹²⁵I) and the like.

Pioglitazone or a salt thereof is particularly preferably pioglitazone hydrochloride.

Pioglitazone or a salt thereof may be diluted with diluent and the like generally used in medicine, food field and the like.

Since powder is generally an aggregate of particles having various sizes, the size of particle is expressed by an average particle size. As the average particle size, median size, modal diameter, arithmetic mean diameter and the like are used. Median size is also indicated as d50, and means a particle size that divides coarse particles and fine granules into 50% each in weight distribution or number distribution. The average particle size used in the present specification is mainly expressed by the median size.

The measurement method of powder includes laser diffraction method, dynamic light scattering method, centrifugal sedimentation method, FFF method, electrical sensing zone method and the like. Examples of the laser diffraction method particle size distribution measuring apparatus include Mastersizer 2000 (manufactured by Malvern), HELOS&RODOS (manufactured by SYMPATEC), SALD2200 (manufactured by Shimadzu Corporation), LA-920 (manufactured by Horiba, Ltd.) and the like. The average particle size described in the present specification is a value measured by Mastersizer 2000 applying the laser diffraction method.

The average particle size of pioglitazone or a salt thereof used as a drug substance in the controlled-release solid preparation of the present invention is generally 1 - 25 µm, preferably 2 - 21 µm, more preferably 2 - 10 µm. Adopting such average particle size, a preparation superior in absorbability can be obtained.

The above-mentioned preferable average particle size is applied to pioglitazone or a salt thereof used as a drug substance (including pulverized products obtained by pulverization in the production process of a controlled-release solid preparation, and the like) for producing the controlled-release solid preparation of the present invention. That is, the average particle size of pioglitazone or a salt thereof may vary beyond the above-mentioned range due to coagulation and the like of pioglitazone or a salt thereof, in the production process of the controlled-release solid preparation of the present invention or preservation process of the controlled-release solid preparation after production. Pulverization is performed using a preparation machine, for example, mortar, jet mill, hammer mill, screen mill and the like.

While the content of pioglitazone or a salt thereof in the controlled-release solid preparation of the present invention varies depending on the dosage form of the controlled-release solid preparation, target disease, severity of disease, and the like, it is generally an amount corresponding to 0.1 - 45 mg, preferably 0.1 - 30 mg, more preferably 0.1 - 15 mg, further preferably 0.1 - 10 mg, further more preferably 0.1 - 8 mg, particularly preferably 0.5 - 8 mg, 0.5 - 6 mg or 0.5 - 1 mg, as pioglitazone per preparation unit. The content is suitable for, for example, the prophylaxis and/or treatment of Alzheimer's disease (particularly, prophylaxis and/or treatment of Alzheimer's dementia). Such preparation may be administered 1 to 3 times (preferably, once) per day.

More specifically, examples of the controlled-release solid preparation of the present invention include a preparation containing pioglitazone in an amount of 0.1 mg, 0.3 mg, 0.5 mg, 0.8 mg, 1 mg, 2.8 mg or 5.2 mg per preparation unit; or a salt of pioglitazone in an amount corresponding to 0.1 mg, 0.3 mg, 0.5 mg, 0.8 mg, 1 mg, 2.8 mg or 5.2 mg as pioglitazone per preparation unit. The preparation is preferably administered once per day.

The content of pioglitazone or a salt thereof in the controlled-release solid preparation of the present invention is, for example, generally 0.05 - 20 wt%, preferably 0.06 - 15 wt%, more preferably 0.08 - 10 wt%, as pioglitazone.

The controlled-release solid preparation of the present invention is preferably a preparation having Cmax of 20 - 140 ng/mL and AUC₀₋₂₄ₕᵣ of 100 - 510 ng/mL, more preferably a preparation having Cmax of 40 - 130 ng/mL and AUC₀₋₂₄ₕᵣ of 160 - 480 ng/mL, further preferably a preparation having Cmax of 60 - 125 ng/mL and AUC₀₋₂₄ₕᵣ of 220 - 450 ng/mL, when pioglitazone is orally administered to a pentagastrin-treated beagle under fasting conditions and kinetics of pioglitazone in blood is measured after the administration, and the area under the blood drug concentration-time curve (AUC₀₋₂₄ₕᵣ) is calculated by the trapezoidal rule based on the measurement results of plasma concentration before administration, and after 0.5, 1, 2, 4, 6, 8, 12 hr and 24 hr, and when 1 mg of pioglitazone, or 1 mg as pioglitazone, is administered. Even when they are outside such ranges, a preparation preferable from the aspect of the below-mentioned dissolution property is encompassed in the preparation of the present invention.

Having such particular feature of the preparation, a preparation having the effects of the aforementioned (1) - (4), and useful for the prophylaxis and/or treatment of Alzheimer's disease (particularly, prophylaxis and/or treatment of Alzheimer's dementia) can be provided.

Examples of the controlled-release solid preparation of the present invention capable of achieving the above-mentioned Cmax and AUC include (I) a controlled-release solid preparation containing pioglitazone or a salt thereof as an active ingredient, which is constituted of an immediate-release part and a sustained-release part in combination, (II) a preparation controlling drug release by osmotic pressure pump, (III) an intragastric floating preparation and the like.

The above-mentioned preparations are explained below.

(I) controlled-release solid preparation containing pioglitazone or a salt thereof as an active ingredient, which is constituted of an immediate-release part and a sustained-release part in combination

A preferable embodiment is a controlled-release solid preparation containing pioglitazone or a salt thereof in an amount of 0.1 - 8 mg (preferably 0.5 - 8 mg, more preferably 0.5 - 6 mg, further preferably 0.5 - 1 mg) as pioglitazone per preparation unit, which is constituted of an immediate-release part and a sustained-release part in combination.

In the present specification, the "sustained-release part" is a part constituting the preparation, and controlling the dissolution of a drug so that the effective blood concentration of the drug can be maintained for a long time after administration of the preparation, and the "immediate-release part" is a part constituting the preparation, which releases the drug immediately after administration of the preparation, or which does not control drug release.

These immediate-release part and sustained-release part may be a two-layer tablet (multi-layer tablet) wherein each layer is adhered to each other or a nucleated tablet comprising the inner core and the outer layer. Furthermore, they can be granulated into a portion pack or capsule.

The preparation includes a matrix two-layer preparation (matrix two-layer tablet), a membrane-controlled granule preparation, and a membrane-controlled sustained-release preparation.

In the preparation, pioglitazone or a salt thereof may be contained in one or both of an immediate-release part and a sustained-release part.

### (i) Matrix two-layer preparation (matrix two-layer tablet)

Examples of the matrix two-layer preparation (matrix two-layer tablet) include a preparation (tablet) having a structure wherein an immediate-release part (immediate-release layer (IR (immediate-release) layer)), and a sustained-release part (sustained-release layer (SR (sustained-release) layer)) are laminated.

In the preparations, pioglitazone or a salt thereof is preferably contained in both an immediate-release part and a sustained-release part.

IR layer contains, for example, pioglitazone or a salt thereof (e.g., pioglitazone hydrochloride) and additive (e.g., excipient, disintegrant, lubricant).

As the additive, additives conventionally used in the below-mentioned technical field of preparation can be used.

The content of pioglitazone or a salt thereof in the IR layer is generally 0.05 - 20 wt%, preferably 0.06 - 15 wt%, more preferably 0.08 - 10 wt%, as pioglitazone, relative to the IR layer.

The content of excipient in the IR layer is generally 30 - 99 wt%, preferably 40 - 98 wt%, more preferably 50 - 95 wt%, relative to the IR layer.

The content of disintegrant in the IR layer is generally 1 - 15 wt%, preferably 3 - 10 wt%, more preferably 4 - 8 wt%, relative to the IR layer.

The content of lubricant in the IR layer is generally 0.01 - 3 wt%, preferably 0.05 - 2 wt%, more preferably 0.1 - 1 wt%, relative to the IR layer.

The SR layer is a layer containing, for example, pioglitazone or a salt thereof (e.g., pioglitazone hydrochloride) and additive (e.g., gel forming promoter, gel forming polymer, antioxidant, lubricant). The SR layer preferably contains a gel forming polymer.

As the additive, those conventionally used in the below-mentioned technical field of preparation can be used.

Examples of the gel forming polymer to be used in the present invention include polyethylene oxide, hypromellose, hydroxypropylcellulose, methylcellulose, sodium carboxymethylcellulose, low-substituted hydroxypropylcellulose, croscarmellose sodium, and the like. Polyethylene oxide is preferable.

In the present specification, a gel forming promoter means an excipient having an action to promote penetration of water into the preparation before gelling of the gel forming polymer, and any excipient having such action is used without particular limitation. In the controlled-release solid preparation of the present invention, a part of the gel forming promoter also has a function as a surface modifier.

In the present specification, a surface modifier is not particularly limited as long as it has an action to accelerate gelling of a gel forming polymer by surface modification of the gel forming polymer.

Examples of the gel forming promoter to be used in the present invention include water-soluble hydrophilic base, and water-insoluble hydrophilic base.

Examples of the water-soluble hydrophilic base to be used in the present invention include sugar and sugar alcohols such as lactose, glucose, mannitol, trehalose, D-sorbitol, xylitol, sucrose, maltose, lactulose, D-fructose, dextran, glucose and the like, water-soluble polymers such as polyethylene glycol (e.g., macrogol 400, macrogol 1500, macrogol 4000, macrogol 6000, macrogol 20000 (all manufactured by NOF Corporation)), polyvinylpyrrolidone (e.g., PVP (registered trade mark) K30 (manufactured by BASF)) and the like, surfactants such as polyoxyethylene hydrogenated castor oil (e.g., Cremophor (registered trade mark) RH40 (manufactured by BASF), HCO-40, HCO-60 (manufactured by Nikko Chemicals)), polyoxyethylenepolyoxypropyleneglycol (e.g., pluronic (registered trade mark) F68 (manufactured by ADEKA CORPORATION) etc.) or sorbitan polyoxyethylene higher fatty acid ester (e.g., Tween80 (manufactured by KANTO KAGAKU) etc.) and the like, salts such as sodium chloride, magnesium chloride and the like, organic acids such as citric acid, tartaric acid and the like, amino acids such as glycine, β-alanine, lysine hydrochloride and the like, amino sugars such as meglumine and the like, and the like. One or more kinds thereof may be used in combination.

Examples of the water-insoluble hydrophilic base to be used in the present invention include starch, cereal flour including starch (e.g., corn starch, potato starch, wheat starch, rice starch), partly pregelatinized starch, hydroxypropylstarch, crospovidone, crystalline cellulose (CEOLUS KG801, KG802, PH101, PH102, PH301, PH302, PH-F20, RC-A591NF, KG1000, PH101D, PH301D, PH301Z, UF702, UF711 (trade name, manufactured by Asahi Kasei Chemicals Corporation), called microcrystalline cellulose), fine particles silicic anhydride (light anhydrous silicic acid free of hydrophobic treatment or amorphous silica fine particles having particle size of 0.1 micron or below), carboxymethylcellulose, carboxymethylcellulose calcium (carmellose calcium), sodium carboxymethyl starch, carmellose sodium, croscarmellose sodium, carmellose, carmellose calcium, low-substituted hydroxypropylcellulose [preferably, low-substituted hydroxypropylcellulose having a hydroxypropoxy group content of 5 - 16 wt% such as LH-11, LH-21, LH-31, LH-22, LH-32, LH-20, LH-30, LH-33, LH-B1, NBD-020, NBD-021, NBD-022 (trade name, manufactured by Shin-Etsu Chemical Co., Ltd.) and the like] and the like, and one or more kinds thereof may be used in combination.

The content of pioglitazone or a salt thereof in the SR layer is generally 0.05 - 20 wt%, preferably 0.06 - 15 wt%, more preferably 0.08 - 10 wt%, as pioglitazone, relative to the SR layer.

The content of a gel forming polymer in the SR layer is generally 5 - 80 wt%, preferably 10 - 60 wt%, more preferably 20 - 40 wt%, relative to the SR layer.

The content of a gel forming promoter in the SR layer is generally 10 - 95 wt%, preferably 30 - 85 wt%, more preferably 50 - 80 wt%, relative to the SR layer.

The content of an antioxidant in the SR layer is generally 0.01 - 3 wt%, preferably 0.03 - 2 wt%, more preferably 0.05 - 1 wt%, relative to the SR layer.

The content of a lubricant in the SR layer is generally 0.01 - 5 wt%, preferably 0.3 - 3 wt%, more preferably 0.5 - 2 wt%, relative to the SR layer.

To achieve the effects of the aforementioned (1) - (4), the weight ratio of pioglitazone or a salt thereof in the IR layer and pioglitazone or a salt thereof in the SR layer is preferably 1:0.1 - 15, more preferably 1:0.1 - 10, further preferably 1:0.3 - 10, further more preferably 1:0.5 - 5, particularly preferably 1:0.5 - 2.

When a salt of pioglitazone is used in the above, the weight of the salt of pioglitazone corresponds to an amount as pioglitazone.

In the present invention, the matrix two-layer preparation (matrix two-layer tablet) can be produced using the above-mentioned various additives and according to a method conventionally used in the technical field of preparation.

For example, the matrix two-layer tablet in the present invention can be produced by the following method.

Pioglitazone or a salt thereof (e.g., pioglitazone hydrochloride), and additive (e.g., excipient, disintegrant, lubricant) are mixed and, where necessary, the mixture is granulated to give granules or a mixture for tableting of IR layer. Separately, pioglitazone or a salt thereof (e.g., pioglitazone hydrochloride), and additive (e.g., gel forming promoter, gel forming polymer, antioxidant, lubricant) are mixed and, where necessary, the mixture is granulated to give granules or a mixture for tableting of SR layer. The obtained granule or mixture for tableting of IR layer is compression molded to form IR layer, the granule or mixture for tableting of SR layer is added on the obtained IR layer and compression molded to form SR layer, whereby a matrix two-layer tablet can be produced.

Mixing can be performed using, for example, a blending machine such as a V-type mixer, a tumbler mixer and the like. Granulation can be performed using, for example, a high speed mixer granulator, a fluid bed dryer granulator and the like. Compression molding can be performed, for example, by tableting using a single punch tableting machine, a rotary tableting machine and the like.

The controlled-release solid preparation of the present invention is preferably a solid preparation (e.g., matrix two-layer tablet) containing pioglitazone or a salt thereof in an amount of 0.1 - 8 mg as pioglitazone per preparation unit, constituted of an immediate-release part and a sustained-release part in combination, and having a structure wherein pioglitazone or a salt thereof is contained in both the immediate-release part and the sustained-release part, and the immediate-release part and the sustained-release part are laminated, and shows the following dissolution pattern:
a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.

### (ii) membrane-controlled granule preparation

Examples of the membrane-controlled granule preparation include a preparation wherein one or each of the immediate-release part and the sustained-release part has a granule structure.

Examples of the preparation include (ii-1) a preparation wherein the both of an immediate-release part and a sustained-release part have a granule structure; (ii-2) a preparation wherein an immediate-release part has a granule structure, and a sustained-release part forms an outer layer covering same; (ii-3) tablet wherein an immediate-release part and a sustained-release part are dispersed; (ii-4) a preparation (e.g., tablet) having a structure wherein an immediate-release part has a granule structure, and a sustained-release part forms an outer layer covering same and a sustained-release part is dispersed in the outer layer.

The above-mentioned preparations (ii-1), (ii-2) are preferable.

Preparation (ii-1) is explained first.

In the above-mentioned preparation, the sustained-release part may be a sustained-release granule wherein an immediate-release granule (IR granule) is coated with a coating layer containing a polymer providing sustained release property (also referred to as SR granule in the present specification), or a sustained-release granule wherein an immediate-release granule is coated with a coating layer containing an enteric polymer (also referred to as DR (Delayed-Release) granule in the present specification). That is, the preparation may be, for example, a preparation constituted of a combination of IR granule and DR granule, or a preparation constituted of a combination of IR granule and SR granule.

Examples of the preparation include a capsule filled with an immediate-release part and a sustained-release part, for example, a capsule filled with IR granule and DR granule, and a capsule filled with IR granule and SR granule.

A preparation constituted of a combination of IR granules and DR granules (e.g., capsule filled with IR granule and DR granule) is explained below.

The IR granule contains, for example, pioglitazone or a salt thereof (e.g., pioglitazone hydrochloride) and additive (e.g., excipient, binder). The IR granule may be a granule wherein a core granule (e.g., Nonpareil (manufactured by FREUND, spherical granulation product of crystalline cellulose and lactose)) is coated with pioglitazone or a salt thereof and additive.

As the additive, the below-mentioned additives conventionally used in the technical field of preparation can be used.

The content of pioglitazone or a salt thereof in the IR granule is generally 0.05 - 20 wt%, preferably 0.06 - 15 wt%, more preferably 0.08 - 10 wt%, as pioglitazone, relative to the IR granule.

The content of an excipient in the IR granule is generally 20 - 90 wt%, preferably 30 - 80 wt%, more preferably 40 - 70 wt%, relative to the IR granule.

The content of a binder in the IR granule is generally 5 - 40 wt%, preferably 8 - 30 wt%, more preferably 10 - 20 wt%, relative to the IR granule.

The content of a core granule in the IR granule is generally 5 - 60 wt%, preferably 10 - 50 wt%, more preferably 20 - 40 wt%, relative to the IR granule.

The DR granule is obtained by coating IR granule with a coating layer containing an enteric polymer.

The coating layer contains, for example, an enteric polymer, and a coating additive to be added optionally (e.g., plasticizer, lubricant).

As the enteric polymer and additive, those conventionally used in the technical field of preparation to be mentioned below can be used.

In the present invention, the enteric polymer is preferably a polymer soluble at preferably pH 3 - pH 9, more preferably pH 4 - pH 8, further preferably pH 5 - pH 7, further more preferably pH 5.5 - 6.5. In the present invention, preferable examples of the enteric polymer include methacrylic acid copolymer L [Eudragit L100 (trade name) manufactured by EVONIK], methacrylic acid copolymer LD [Eudragit L-30D55 (trade name) manufactured by EVONIK], and methacrylic acid copolymer S [Eudragit S100 (trade name) manufactured by EVONIK].

In the present invention, the DR granule is preferably soluble at preferably pH 3 - pH 9, more preferably pH 4 - pH 8, further preferably pH 5 - pH 7, further more preferably pH 5.5 - 6.5.

In the present invention, the coating layer containing an enteric polymer preferably shows dissolution property at preferably pH 3 - pH 9, more preferably pH 4 - pH 8, further preferably pH 5 - pH 7, further more preferably pH 5.5 - pH 6.5.

The content of an enteric polymer in the coating layer of DR granule is generally 3 - 50 wt%, preferably 5 - 40 wt%, more preferably 10 - 30 wt%, relative to the core granules.

The content of a plasticizer in the coating layer of DR granule is generally 3 - 70 wt%, preferably 5 - 60 wt%, more preferably 10 - 50 wt%, relative to the enteric polymer.

The content of a lubricant in the coating layer of DR granule is generally 10 - 70 wt%, preferably 20 - 60 wt%, more preferably 30 - 50 wt%, relative to the enteric polymer.

In the preparation, pioglitazone or a salt thereof is preferably contained in each of the immediate-release part and the sustained-release part.

To achieve the effects of the aforementioned (1) - (4), the weight ratio of pioglitazone or a salt thereof in IR granule, and pioglitazone or a salt thereof in DR granule is preferably 1:0.1 - 15, more preferably 1:0.1 - 10, further preferably 1:0.1 - 5, further more preferably 1:0.1 - 3, particularly preferably 1:0.5 - 1.

When a salt of pioglitazone is used in the above, the weight of the salt of pioglitazone corresponds to an amount as pioglitazone.

Next, a preparation constituted of a combination of IR granule and SR granule (e.g., capsule filled with IR granule and SR granule) is explained.

IR granule is similar to those explained with regard to the above-mentioned preparation constituted of a combination of IR granule and DR granule.

The SR granule is obtained by coating the IR granule with a coating layer containing a sustained-release polymer (also referred to as SR polymer in the present specification).

The coating layer contains, for example, an insoluble polymer, a water-soluble polymer, and a coating additive to be added optionally (e.g., plasticizer, lubricant).

As the insoluble polymer, water-soluble polymer, and additive, those conventionally used in the technical field of preparation to be mentioned later can be used.

As the insoluble polymer in the present invention, cellulose polymers such as ethylcellulose, cellulose acetate and the like; acrylic acid polymers such as aminoalkylmethacrylate copolymer RS [Eudragit RS (trade name) manufactured by EVONIK], ethyl acrylate-methyl methacrylate copolymer suspension [Eudragit NE (trade name) manufactured by EVONIK] and the like; vinyl polymers such as polyvinyl acetate dispersion [Kollicoat SR30D (trade name) manufactured by BASF] and the like, and the like are preferable. Among these, Kollicoat SR30D (Kollicoat SR 30D) is preferable.

As the water-soluble polymer in the present invention, cellulose polymers such as hydroxypropylcellulose, hydroxypropylmethylcellulose, hydroxyethylcellulose, methylhydroxyethylcellulose and the like; polyvinylacetal diethylaminoacetate, aminoalkyl methacrylate copolymer E [Eudragit E (trade name) manufactured by EVONIK], polyvinylpyrrolidone; synthetic polymers such as polyvinyl alcohol-polyethylene glycol graft copolymer [Kollicoat IR (trade name) manufactured by BASF] and the like; polysaccharides such as pullulan and the like, and the like are preferable. Among these, Kollicoat IR (Kollicoat IR) is preferable.

The content of an insoluble polymer in the coating layer of SR granule is generally 3 - 40 wt%, preferably 5 - 30 wt%, more preferably 10 - 20 wt%, relative to the core granules. The content of a water-soluble polymer in the coating layer of SR granule is generally 5 - 90 wt%, preferably 10 - 80 wt%, more preferably 20 - 70 wt%, relative to the total amount of the polymer.

The content of a plasticizer in the coating layer of SR granule is generally 1 - 40 wt%, preferably 2 - 30 wt%, more preferably 3 - 20 wt%, relative to the total amount of the polymer.

The content of a lubricant in the coating layer of SR granule is generally 10 - 70 wt%, preferably 20 - 60 wt%, more preferably 30 - 50 wt%, relative to the total amount of the polymer.

In the preparation, pioglitazone or a salt thereof is preferably contained in each of the immediate-release part and the sustained-release part.

To achieve the effects of the aforementioned (1) - (4), the weight ratio of pioglitazone or a salt thereof in IR granule, and pioglitazone or a salt thereof in SR granule is preferably 1:0.1 - 20, more preferably 1:0.1 - 15, more preferably 1:0.3 - 5, further more preferably 1:0.3 - 3, particularly preferably 1:0.5 - 1.

When a salt of pioglitazone is used, the weight of the salt of pioglitazone corresponds to an amount as pioglitazone.

The membrane-controlled granule preparation of the present invention (e.g., capsule filled with IR granule and DR granule or capsule filled with IR granule and SR granule) can be produced using the above-mentioned various additives and according to a method conventionally used in the technical field of preparation.

For example, a capsule filled with IR granule and DR granule in the present invention can be produced by the following method.

Pioglitazone or a salt thereof (e.g., pioglitazone hydrochloride) and additive (e.g., excipient, binder) are mixed, the obtained mixture and binder (e.g., hydroxypropylcellulose) are sprayed on a core granule, and dried to give IR granule. Separately, a solution of enteric polymer and additive (e.g., plasticizer, lubricant) or a dispersion thereof is sprayed on IR granule obtained in the same manner as above for granulation, which is followed by drying to give DR granule. The obtained IR granule and DR granule are filled in a capsule, whereby the capsule of the present invention can be produced.

Mixing and granulation can be performed using the above-mentioned blending machine and granulating machine.

A capsule filled with IR granule and SR granule in the present invention can be produced by, for example, a method similar to the above-mentioned method except that an insoluble polymer and a water-soluble polymer are used instead of the enteric polymer.

The controlled-release solid preparation of the present invention is preferably a solid preparation (e.g., capsule filled with IR granule and DR granule) containing pioglitazone or a salt thereof in an amount of 0.1 - 8 mg as pioglitazone per preparation unit, constituted of an immediate-release part and a sustained-release part in combination, and having a structure wherein pioglitazone or a salt thereof is contained in one or each of the immediate-release part and the sustained-release part, and each of the immediate-release part and the sustained-release part has a granule structure, and shows the following dissolution pattern:
a dissolution rate of pioglitazone of 10-90% on average at 2 hr time point and 30-110% on average at 4 hr time point, preferably 20-80% on average at 2 hr time point and 40-110% on average at 4 hr time point, in a dissolution test comprising dropping one capsule obtained by filling granules constituting the immediate-release part (IR granule) and granules constituting the sustained-release part (DR granule) in 900 mL of a hydrochloric acid solution (pH 1.2) and evaluating same by the USP basket method at rotation number of 100 rpm, 37°C and, 2 hr after dropping the capsule, changing the test solution to 900 mL of a phosphate buffer (pH 6.8)/0.1% CTAB solution and evaluating same under similar conditions.

Next, preparation (ii-2) is explained.

A preparation wherein the immediate-release part has a granule structure, and the sustained-release part constitutes an outer layer covering the granule structure is, for example, a preparation wherein the immediate-release part has a granule structure, and the sustained-release part is applied as a coating layer to the granule structure.

In the preparation, the sustained-release part may be a coating layer containing a polymer providing sustained release property (sustained-release polymer), or a coating layer containing an enteric polymer. That is, the preparation may be, for example, a preparation having a granule structure wherein an immediate-release granule (IR granule) is coated with a coating layer containing a sustained-release polymer, or a preparation having a granule structure wherein IR granule is coated with a coating layer containing an enteric polymer.

Examples of the preparation include a capsule filled with an immediate-release part and a sustained-release part, for example, a capsule filled with granules obtained by coating IR granule with a coating layer containing a sustained-release polymer, a capsule filled with granules obtained by coating IR granule with a coating layer containing an enteric polymer.

IR granule is similar to those explained with regard to the above-mentioned preparation (ii-1).

In the preparation, pioglitazone or a salt thereof is preferably contained only in an immediate-release part (IR granule).

The "coating layer containing a sustained-release polymer" is, for example, a layer containing an insoluble polymer, a water-soluble polymer, and a coating additive that may be added optionally (e.g., plasticizer, lubricant), and examples thereof include those similar to the ones explained for the above-mentioned preparation (ii-1).

As the sustained-release polymer, a polymer composed of a combination of a water-soluble polymer and an insoluble polymer is preferable. Preferred as the water-soluble polymer is Kollicoat IR. Preferred as the insoluble polymer is Kollicoat SR30D.

The content of an insoluble polymer in the coating layer is generally 1 - 25 wt%, preferably 5 - 20 wt%, more preferably 10 - 15 wt%, relative to the core granules.

The content of a water-soluble polymer in the coating layer is generally 5 - 50 wt%, preferably 10 - 40 wt%, more preferably 20 - 30 wt%, relative to the total amount of the polymer.

The content of a plasticizer in the coating layer is generally 1 - 10 wt%, preferably 3 - 7 wt%, relative to the total amount of the polymer.

The content of a lubricant in the coating layer is generally 0.5 - 15 wt%, preferably 1 - 10 wt%, more preferably 3 - 8 wt%, relative to the total amount of the polymer.

The amount of the coating layer to be coated is generally 1 - 30 wt%, preferably 5 - 15 wt%, relative to the core granules.

The "coating layer containing an enteric polymer" is, for example, a layer containing an enteric polymer and a coating additive that may be added optionally (e.g., plasticizer, lubricant), and examples thereof include those similar to the ones explained for the above-mentioned preparation (ii-1).

The content of an enteric polymer in the coating layer is generally 3 - 50 wt%, preferably 5 - 40 wt%, more preferably 10 - 30 wt%, relative to the core granules.

The content of a plasticizer in the coating layer is generally 3 - 70 wt%, preferably 5 - 60 wt%, more preferably 10 - 50 wt%, relative to the enteric polymer.

The content of a lubricant in the coating layer is generally 10 - 70 wt%, preferably 20 - 60 wt%, more preferably 30 - 50 wt%, relative to the enteric polymer.

The amount of the coating layer to be coated is generally 1 - 30 wt%, preferably 5 - 15 wt%, relative to the core granules.

The membrane-controlled sustained-release preparation of the present invention (e.g., capsule filled with granules obtained by coating IR granule with a coating layer containing a sustained-release polymer or capsule filled with granules obtained by coating IR granule with a coating layer containing an enteric polymer) can be produced using the above-mentioned various additives and according to a method conventionally used in the technical field of preparation.

For example, a capsule filled with granules obtained by coating IR granule with a coating layer containing a sustained-release polymer and a capsule filled with granules obtained by coating IR granule with a coating layer containing an enteric polymer in the present invention can be produced by the following method.

Pioglitazone or a salt thereof (e.g., pioglitazone hydrochloride) and additive (e.g., excipient, binder) are mixed, the obtained mixture and binder (e.g., hydroxypropylcellulose) are sprayed on a core granule, and dried to give IR granule. The obtained IR granule is coated with the above-mentioned coating components to give granules. The obtained granules are filled in a capsule, whereby the capsule of the present invention can be produced.

Mixing and granulation can be performed using the above-mentioned blending machine and granulating machine.

In the controlled-release solid preparation of the present invention, a solid preparation (e.g., capsule filled with granules obtained by coating IR granule with a coating layer containing a sustained-release polymer) containing pioglitazone or a salt thereof in an amount of 0.1 - 8 mg as pioglitazone per preparation unit, constituted of an immediate-release part and a sustained-release part in combination, and having a granule structure wherein pioglitazone or a salt thereof is contained in one or each of the immediate-release part and the sustained-release part, the immediate-release part has a granule structure, and the sustained-release part is applied as a coating layer to the granule structure preferably shows the following dissolution pattern:
a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP basket method at 37°C, with rotation number of 100 rpm using a 0.3M potassium chloride buffer (pH 2) as a test solution.

### (iii) membrane-controlled sustained-release preparation

Examples of the membrane-controlled sustained-release preparation include a preparation having a structure wherein one of the immediate-release part and the sustained-release part constitutes an inner core and the other constitutes an outer layer covering the inner core.

Examples of the preparation include (iii-1) a preparation wherein the inner core has a tablet structure, the outer layer has a membrane structure, and (iii-2) a preparation wherein the inner core has a tablet structure. Examples of these preparations include a tablet.

First, preparation (iii-1) is explained.

Examples of the preparation include a preparation (e.g., tablet) having a structure wherein an immediate-release part constitutes the inner core (also referred to as IR tablet in the present specification), a sustained-release part constitutes an outer layer covering the same (also referred to as SR coating in the present specification), and the outer layer is a coating layer containing a water-soluble polymer and an insoluble polymer.

A tablet wherein an immediate-release part constitutes IR tablet, and a sustained-release part constitutes SR coating covering the same is explained below.

The IR tablet is a tablet containing, for example, pioglitazone or a salt thereof (e.g., pioglitazone hydrochloride) and additive (e.g., excipient, disintegrant, binder, lubricant).

As the additive, the below-mentioned additives conventionally used in the technical field of preparation can be used.

The content of pioglitazone or a salt thereof in the IR tablet is generally 0.05 - 20 wt%, preferably 0.06 - 15 wt%, more preferably 0.08 - 10 wt%, relative to the IR tablet.

The content of an excipient in the IR tablet is generally 30 - 90 wt%, preferably 40 - 80 wt%, more preferably 50 - 70 wt%, relative to the IR tablet.

The content of a disintegrant in the IR tablet is generally 5 - 60 wt%, preferably 10 - 50 wt%, more preferably 20 - 40 wt%, relative to the IR tablet.

The content of a binder in the IR tablet is generally 0.5 - 20 wt%, preferably 1 - 10 wt%, more preferably 2 - 5 wt%, relative to the IR tablet.

The content of a lubricant in the IR tablet is generally 0.05 - 5 wt%, preferably 0.1 - 3 wt%, more preferably 0.5 - 1 wt%, relative to the IR tablet.

Pioglitazone or a salt thereof is preferably contained in only an immediate-release part (IR tablet) in the preparation.

The SR coating layer contains, for example, an insoluble polymer, a water-soluble polymer, and a coating additive to be added optionally (e.g., plasticizer, lubricant).

As the insoluble polymer, water-soluble polymer, and additive, those conventionally used in the technical field of preparation to be mentioned later can be used.

In the present invention, preferred as the insoluble polymer is Kollicoat SR 30D. In the present invention, preferred as the water-soluble polymer is Kollicoat IR.

The content of an insoluble polymer in the SR coating layer is generally 1 - 30 wt%, preferably 2 - 20 wt%, more preferably 3 - 10 wt%, relative to the IR tablet. The content of a water-soluble polymer in the SR coating layer is generally 5 - 90 wt%, preferably 10 - 90 wt%, more preferably 20 - 70 wt%, relative to the total amount of the polymer.

The content of a plasticizer in the SR coating layer is generally 2 - 40 wt%, preferably 3 - 30 wt%, more preferably 5 - 20 wt%, relative to the total amount of the polymer.

The content of a lubricant in the SR coating layer is generally 10 - 70 wt%, preferably 20 - 60 wt%, more preferably 30 - 50 wt%, relative to the total amount of the polymer.

The amount of the coating layer to be applied is generally 1 - 30 wt%, preferably 2 - 20 wt%, relative to the core tablet.

The membrane-controlled sustained-release preparation (e.g., tablet containing IR tablet and SR coating covering same) of the present invention can be produced using the above-mentioned various additives and according to a method conventionally used in the technical field of preparation.

For example, the tablet containing IR tablet and SR coating covering same of the present invention can be produced by the following method.

Pioglitazone or a salt thereof (e.g., pioglitazone hydrochloride) and additive (e.g., excipient, disintegrant) are mixed, a solution of a binder (e.g., hydroxypropylcellulose) is sprayed for granulation, followed by drying and, where necessary, sieving to obtain granules, with which additive (e.g., disintegrant, lubricant) is mixed and the mixture is compression molded to give IR tablet. The obtained IR tablet is coated with the above-mentioned coating components, whereby the tablet of the present invention can be produced.

Mixing, granulation and compression molding can be performed using the above-mentioned blending machine, granulating machine and tableting machine.

In the controlled-release solid preparation of the present invention, a solid preparation (e.g., tablet containing IR tablet and SR coating covering same) containing pioglitazone or a salt thereof in an amount of 0.1 - 8 mg as pioglitazone per preparation unit, constituted of an immediate-release part and a sustained-release part in combination, and having a structure wherein pioglitazone or a salt thereof is contained in one or each of the immediate-release part and the sustained-release part, one of the immediate-release part and the sustained-release part constitutes the inner core, and the other constitutes an outer layer covering same, the inner core has a tablet structure, and the outer layer has a membrane structure preferably shows the following dissolution pattern:
a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.

Now, preparation (iii-2) is explained.

Examples of the preparation include a preparation (e.g., tablet) having a structure wherein a sustained-release part constitutes an inner core (also referred to as SR layer in the present specification), and an immediate-release part constitutes an outer layer covering same (referred to as IR layer in the present specification).

In the preparations, pioglitazone or a salt thereof is preferably contained in both an immediate-release part and a sustained-release part.

The SR layer is a layer containing, for example, pioglitazone or a salt thereof (e.g., pioglitazone hydrochloride) and additive (e.g., gel forming promoter, gel forming polymer, antioxidant, lubricant). The SR layer preferably contains a gel forming polymer.

As the additive, the below-mentioned additives conventionally used in the technical field of preparation can be used.

As the "gel forming polymer" and "gel forming promoter", examples thereof include those similar to the ones explained for the above-mentioned matrix two-layer preparation. As the gel forming polymer, polyethylene oxide is preferable.

The content of pioglitazone or a salt thereof in the SR layer is generally 0.05 - 20 wt%, preferably 0.06 - 15 wt%, more preferably 0.08 - 10 wt%, as pioglitazone, relative to the SR layer.

The content of a gel forming polymer in the SR layer is generally 5 - 80 wt%, preferably 10 - 60 wt%, more preferably 20 - 40 wt%, relative to the SR layer.

The content of a gel forming promoter in the SR layer is generally 10 - 95 wt%, preferably 30 - 85 wt%, more preferably 50 - 80 wt%, relative to the SR layer.

The content of an antioxidant in the SR layer is generally 0.01 - 3 wt%, preferably 0.03 - 2 wt%, more preferably 0.05 - 1 wt%, relative to the SR layer.

The content of a lubricant in the SR layer is generally 0.01 - 5 wt%, preferably 0.3 - 3 wt%, more preferably 0.5 - 2 wt%, relative to the SR layer.

The IR layer contains, for example, pioglitazone or a salt thereof (e.g., pioglitazone hydrochloride) and additive (e.g., excipient, disintegrant, lubricant).

As the additive, the below-mentioned conventional additives used in the technical field of preparation can be used.

The content of pioglitazone or a salt thereof in the IR layer is generally 0.05 - 20 wt%, preferably 0.06 - 15 wt%, more preferably 0.08 - 10 wt%, as pioglitazone, relative to the IR layer.

The content of an excipient in the IR layer is generally 30 - 99 wt%, preferably 40 - 98 wt%, more preferably 50 - 95 wt%, relative to the IR layer.

The content of a disintegrant in the IR layer is generally 1 - 15 wt%, preferably 3 - 10 wt%, more preferably 4 - 8 wt%, relative to the IR layer.

The content of a lubricant in the IR layer is generally 0.01 - 3 wt%, preferably 0.05 - 2 wt%, more preferably 0.1 - 1 wt%, relative to the IR layer.

To achieve the effects of the aforementioned (1) - (4), the weight ratio of pioglitazone or a salt thereof in the IR layer and pioglitazone or a salt thereof in the SR layer is preferably 1:0.1 - 15, more preferably 1:0.1 - 10, further preferably 1:0.1 - 5, further more preferably 1:0.1 - 3, particularly preferably 1:0.5 - 2.

When a salt of pioglitazone is used in the above, the weight of the salt of pioglitazone corresponds to an amount as pioglitazone.

The nucleated tablet (e.g., tablet having a structure wherein sustained-release part constitutes SR layer, and immediate-release part constitutes IR layer covering same) of the present invention can be produced using the above-mentioned various additives and according to a method conventionally used in the technical field of preparation.

For example, a tablet having a structure wherein a sustained-release part constitutes SR layer, and an immediate-release part constitutes IR layer covering same can be produced by the following method.

Pioglitazone or a salt thereof (e.g., pioglitazone hydrochloride), and additive (e.g., excipient, disintegrant, lubricant) are mixed and, where necessary, the mixture is granulated to give granules or a mixture for tableting of IR layer. Separately, pioglitazone or a salt thereof (e.g., pioglitazone hydrochloride), and additive (e.g., gel forming promoter, gel forming polymer, antioxidant, lubricant) are mixed and, where necessary, the mixture is granulated to give granules or a mixture for tableting of SR layer. The obtained granules or mixture for tableting of SR layer are/is compression molded to form SR layer (SR tablet). A part of the obtained granules or mixture for tableting of IR layer is weakly tableted, SR layer (SR tablet) is placed to be the center, and the remaining IR layer is added and tableted to form IR layer, whereby the object tablet can be produced.

Mixing, granulation and compression molding can be performed using the above-mentioned blending machine, granulating machine and tableting machine.

In the controlled-release solid preparation of the present invention, a solid preparation (e.g., tablet having a structure wherein sustained-release part constitutes SR layer, and immediate-release part constitutes IR layer covering same) containing pioglitazone or a salt thereof in an amount of 0.1 - 8 mg as pioglitazone per preparation unit, constituted of an immediate-release part and a sustained-release part in combination, and having a structure wherein pioglitazone or a salt thereof is contained in one or each of the immediate-release part and the sustained-release part, one of the immediate-release part and the sustained-release part constitutes the inner core, and the other constitutes an outer layer covering same, and the inner core has a tablet structure preferably shows the following dissolution pattern:
a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.

Besides the above-mentioned, as "a preparation constituted of an immediate-release part and a sustained-release part in combination, and having a structure wherein one of the immediate-release part and the sustained-release part constitutes the inner core, and the other constitutes an outer layer covering same", a preparation having a structure wherein the inner core has a layer structure wherein a highly swellable polymer layer and one or plural immediate-release layers containing pioglitazone or a salt thereof are laminated, the inner core is coated with a semipermeable membrane outer layer, and a semipermeable membrane on the drug-containing immediate-release layer side of the inner core has a minute hole for drug release, and the immediate-release part constitutes an outer layer covering the inner core; and the like can be mentioned.

In the above-mentioned preparation, the outer layer may have a membrane structure (preferably, coating layer composed of water-soluble polymer and insoluble polymer).

The above-mentioned preparation can be produced by a method conventionally used in the technical field of preparation.

### (II) Preparation controlling drug release by osmotic pressure pump

Examples of the preparation include a controlled-release solid preparation (preferably tablet) having a structure wherein the inner core has a layer structure wherein a highly swellable polymer layer and one or plural immediate-release layers containing pioglitazone or a salt thereof are laminated, the inner core is coated with a semipermeable membrane outer layer, and a semipermeable membrane on the drug-containing immediate-release layer side of the inner core has a minute hole for drug release.

A preferable embodiment is a controlled-release solid preparation (preferably tablet) having a structure wherein the inner core has a layer structure wherein a highly swellable polymer layer and one or plural immediate-release layers containing pioglitazone or a salt thereof in an amount of 0.1 - 8 mg (preferably 0.5 - 8 mg, more preferably 0.5 - 6 mg, further preferably 0.5 - 1 mg) as pioglitazone per preparation unit are laminated, the inner core is coated with a semipermeable membrane outer layer, and a semipermeable membrane on the drug-containing immediate-release layer side of the inner core has a minute hole for drug release.

As used herein, "the inner core" refers to a part to be the center of a preparation constituted of plural structures, which may take a different form depending on the dosage form.

The above-mentioned preparation can be produced by a method conventionally used in the technical field of preparation (e.g., JP-A-2007-506774).

The preparation is a preparation having an inner core containing, for example, pioglitazone or a salt thereof (e.g., pioglitazone hydrochloride) and an additive (e.g., excipient, emulsifier, binder, lubricant), and the inner core is coated with an additive (e.g., water-soluble polymer, insoluble polymer, plasticizer).

As the additive, additives conventionally used in the technical field of preparation to be mentioned later can be used.

The content of pioglitazone or a salt thereof in the preparation is generally 0.05 - 20 wt%, preferably 0.06 - 15 wt%, more preferably 0.08 - 10 wt%, as pioglitazone, relative to the preparation.

The content of an excipient in the preparation is generally 5 - 95 wt%, preferably 10 - 90 wt%, more preferably 20 - 85 wt%, relative to the preparation.

The content of an emulsifier in the preparation is generally 1 - 15 wt%, preferably 3 - 10 wt%, more preferably 4 - 8 wt%, relative to the preparation.

The content of a binder in the preparation is generally 1 - 15 wt%, preferably 3 - 10 wt%, more preferably 4 - 8 wt%, relative to the preparation.

The content of a lubricant in the preparation is generally 0.01 - 3 wt%, preferably 0.05 - 2 wt%, more preferably 0.1 - 1 wt%, relative to the preparation.

The content of a water-soluble polymer in the preparation is generally 0.05 - 10 wt%, preferably 0.5 - 5 wt%, more preferably 1 - 3 wt%, relative to the preparation.

The content of an insoluble polymer in the preparation is generally 0.05 - 20 wt%, preferably 0.5 - 10 wt%, more preferably 1 - 5 wt%, relative to the preparation.

The content of a plasticizer in the preparation is generally 0.01 - 10 wt%, preferably 0.05 - 5 wt%, more preferably 0.1 - 1 wt%, relative to the preparation.

When a salt of pioglitazone is used, the weight of the salt of pioglitazone corresponds to an amount as pioglitazone.

The above-mentioned preparation of the present invention can be produced by a method conventionally used in the technical field of preparation and using the above-mentioned various additives.

For example, the above-mentioned preparation of the present invention can be produced by the following method.

Pioglitazone or a salt thereof (e.g., pioglitazone hydrochloride) and additive (e.g., excipient, emulsifier, binder, lubricant) are mixed and, where necessary, the mixture is granulated to give granules or a mixture for tableting of IR tablet. The granules or a mixture for tableting is/are compression molded (tableted) to give IR tablet, which is coated with a film coating (e.g., water-soluble polymer), and further coated with SR coating (e.g., plasticizer, insoluble polymer) to form a semipermeable thin membrane. Minute holes are formed on the obtained tablet thin membrane (e.g., laser drill), whereby the object preparation can be produced.

Examples of the preparation also include the following embodiments.

One or plural immediate-release layers containing pioglitazone or a salt thereof (also referred to as the drug layer in the present specification) is/are layer(s) containing, for example, pioglitazone or a salt thereof (e.g., pioglitazone hydrochloride) and an additive (e.g., gel forming polymer, lubricant).

The highly swellable polymer layer (also referred to as PUSH layer in the present specification) is a layer containing, for example, a gel forming polymer, a gel forming promoter, and a lubricant.

The inner core of the preparation may be obtained by tableting a mixture of each component of the drug layer, adding a mixture of each component of the PUSH layer to the obtained drug layer, tableting the mixture, and laminating the drug layer and the PUSH layer.

Specific examples of the semipermeable outer film layer include a semipermeable membrane formed by applying Opadry CA (manufactured by Colorcon) solution and drying same.

As the additive, those conventionally used in the technical field of preparation to be mentioned later can be used.

As the gel forming polymer in the drug layer, polyethylene oxide (e.g., Polyox N-80, manufactured by Dow) is preferable. As the gel forming polymer in the PUSH layer, polyethylene oxide (e.g., Polyox Coagulant, manufactured by Dow) is preferable.

The content of pioglitazone or a salt thereof in the drug layer is generally 0.05 - 20 wt%, preferably 0.06 - 15 wt%, more preferably 0.08 - 10 wt%, as pioglitazone, relative to the drug layer.

The content of a gel forming polymer in the drug layer is generally 30 - 99 wt%, preferably 40 - 99 wt%, more preferably 50 - 99 wt%, relative to the drug layer.

The content of a lubricant in the drug layer is generally 0.01 - 3 wt%, preferably 0.05 - 2 wt%, more preferably 0.1 - 1 wt%, relative to the drug layer.

The content of a gel forming polymer in the PUSH layer is generally 5 - 90 wt%, preferably 10 - 80 wt%, more preferably 20 - 70 wt%, relative to the PUSH layer.

The content of a gel forming promoter in the PUSH layer is generally 10 - 95 wt%, preferably 30 - 85 wt%, more preferably 50 - 80 wt%, relative to the PUSH layer.

The content of a lubricant in the PUSH layer is generally 0.01 - 5 wt%, preferably 0.3 - 3 wt%, more preferably 0.5 - 2 wt%, relative to the PUSH layer.

When a salt of pioglitazone is used, the weight of the salt of pioglitazone corresponds to an amount as pioglitazone.

For example, the above-mentioned preparation of the present invention can be produced by the following method.

Pioglitazone or a salt thereof (e.g., pioglitazone hydrochloride) and additive (e.g., gel forming polymer, lubricant) are mixed and, where necessary, the mixture is granulated to give granules or a mixture for tableting of a drug layer (IR layer). A gel forming polymer, a gel forming promoter, and a lubricant are mixed and, where necessary, the mixture is granulated to give granules or a mixture for tableting of PUSH layer. The obtained granules or a mixture for tableting of a drug layer are/is compression molded to form a drug layer, granules or a mixture for tableting the PUSH layer are/is added on the obtained drug layer and the mixture is compression molded to form a PUSH layer to give an inner core (core tablet). The obtained inner core is filled in a capsule for one tablet, immersed in Opadry CA solution dissolved in acetone-water mixed solution and dried. Using an injection needle and the like, a hole is made on the top on the drug side of the capsule, whereby the object preparation can be produced.

### (III) Intragastric floating preparation

Examples of the intragastric floating preparation include a controlled-release solid preparation comprising a water-swellable gel forming polymer, a water-expandable foaming agent component, and pioglitazone or a salt thereof.

Specifically, it is a controlled-release solid preparation comprising a water-swellable gel forming polymer, a water-expandable foaming agent component, and pioglitazone or a salt thereof in an amount of 0.1 - 8 mg (preferably 0.5 - 8 mg, more preferably 0.5 - 6 mg, further preferably 0.5 - 1 mg) as pioglitazone per preparation unit.

Examples of the above-mentioned intragastric floating preparation include (1) a preparation having a matrix structure wherein the water-swellable gel forming polymer, the water-expandable foaming agent component, and pioglitazone or a salt thereof are dispersed (hereinafter to be referred to as intragastric floating preparation (1)), and (2) a preparation comprising (A) a volume swelling preparation part containing the water-expandable foaming agent component, and (B) a preparation part containing the water-swellable gel forming polymer and pioglitazone or a salt thereof, which is laminated on the preparation part (A) or embedded in the preparation part (A) such that a part thereof is exposed (hereinafter to be referred to as intragastric floating preparation (2)).

The intragastric floating preparations (1) and (2) preferably further contain a gel forming promoter.

The intragastric floating preparation (1) may further contain the below-mentioned additives (e.g., lubricant) conventionally used in the technical field of preparation.

In the intragastric floating preparation (2), the part (A) may further contain the below-mentioned additives (e.g., lubricant) conventionally used in the technical field of preparation.

In the intragastric floating preparation (2), the part (B) may be in the dosage form of a solid preparation (e.g., tablet, granule) containing the below-mentioned additives conventionally used in the technical field of preparation.

In the intragastric floating preparation of the present invention (particularly, intragastric floating preparations (1) and (2)), examples of the water-swellable gel forming polymer include polyethylene oxide, hypromellose, hydroxypropylcellulose, methylcellulose, sodium carboxymethylcellulose, low-substituted hydroxypropylcellulose, croscarmellose sodium and the like, with preference given to polyethylene oxide. Examples of polyethylene oxide include Polyox N-12K (manufactured by Dow).

In the intragastric floating preparation of the present invention (particularly, intragastric floating preparations (1) and (2)), examples of the water-expandable foaming agent include hydrogencarbonate, and a combination of hydrogencarbonate and organic acid. As hydrogencarbonate, sodium hydrogen carbonate is preferable. Examples of the organic acid include tartaric acid, succinic acid, and citric acid, with preference given to citric acid.

In the intragastric floating preparation of the present invention (particularly, intragastric floating preparations (1) and (2)), examples of the gel forming promoter include those similar to the gel forming promoters explained with regard to the above-mentioned matrix two-layer preparation, with preference given to crystalline cellulose and D-mannitol.

In the intragastric floating preparation of the present invention (particularly, intragastric floating preparations (1) and (2)), the content of each component is as described below.

The content of pioglitazone or a salt thereof is generally 0.05 - 20 wt%, preferably 0.06 - 15 wt%, more preferably 0.08 - 10 wt%, as pioglitazone, relative to the preparation.

The content of a water-swellable gel forming polymer is generally 5 - 90 wt%, preferably 10 - 70 wt%, more preferably 20 - 50 wt%, relative to the preparation.

The content of a gel forming promoter is generally 5 - 80 wt%, preferably 10 - 60 wt%, more preferably 20 - 40 wt%, relative to the preparation.

The content of a water-expandable foaming agent component is generally 3 - 70 wt%, preferably 5 - 60 wt%, more preferably 10 - 50 wt%, relative to the preparation.

The content of a lubricant is generally 0.01 - 5 wt%, preferably 0.1 - 3 wt%, more preferably 0.5 - 2 wt%, relative to the preparation.

The intragastric floating preparation of the present invention can be produced by a method conventionally used in the technical field of preparation and using the above-mentioned various additives.

For example, pioglitazone or a salt thereof (e.g., pioglitazone hydrochloride), a water-swellable gel forming polymer, a water-expandable foaming agent component, a gel forming promoter, and an additive (e.g., lubricant) are mixed and compression molded, whereby the tablet of the present invention can be produced.

The preparation may be further coated with a water permeable, elastic coating film made from a water-insoluble polymer.

Mixing and compression molding can be performed using the above-mentioned blending machine and tableting machine.

In the controlled-release solid preparation of the present invention, a controlled-release solid preparation (intragastric floating preparation) comprising a water-swellable gel forming polymer, a water-expandable foaming agent component, and pioglitazone or a salt thereof in an amount of 0.1 - 8 mg as pioglitazone per preparation unit preferably shows the following dissolution pattern:
a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.

The controlled-release solid preparation of the present invention may also contain an additive conventionally used in the technical field of preparation. Examples of the additive include excipient, disintegrant, binder, lubricant, colorant, pH adjuster, surfactant, stabilizer, corrigent, sweetener, flavor, fluidizer, antistatic agent, light shielding agent, antioxidant, reducing agent, chelating agent and the like. These additives are used in amounts conventionally employed in the technical field of preparation. Two or more kinds of these additives may be mixed at an appropriate ratio.

Examples of the excipient include saccharides; crystalline cellulose; starches such as corn starch, potato starch, wheat starch, rice starch, partly pregelatinized starch, pregelatinized starch, porous starch, dextrin, carboxymethyl starch and the like; anhydrous calcium phosphate, precipitated calcium carbonate, calcium silicate, powder cellulose, gelatin, light anhydrous silicic acid, synthesis aluminum silicate, magnesium alumino metasilicate, magnesium oxide, calcium phosphate, calcium carbonate, and calcium sulfate.

Examples of the saccharides include sugar, starch sugar, lactose, honey, and sugar alcohol. Two or more kinds of these saccharides may be mixed and used at an appropriate ratio.

Examples of the sugar include sucrose, sucrose, glucosyl sucrose [coupling sugar (trade name)], fructo-oligosaccharide, and palatinose.

Examples of the starch sugar include glucose, malt sugar, powder candy, starch syrup, fructose, and trehalose.

Examples of the lactose include lactose, isomerized lactose (lactulose), and reduced lactose (lactitol).

Examples of the honey include various kinds of honey generally used for eating.

Examples of the sugar alcohol include sorbitol, mannitol (particularly, D-mannitol), maltitol, reduced starch saccharification product, xylitol, reduced paratinose, and erythritol.

The saccharides are preferably sugar alcohol, starch sugar and sucrose, more preferably mannitol, trehalose and sucrose. Among these, mannitol and trehalose are preferable.

Examples of the crystalline cellulose include CEOLUS KG801, KG802, PH101, PH102, PH301, PH302, PH-F20, and RC-A591NF (trade name, manufactured by Asahi Kasei Chemicals Corporation), and also include those called microcrystalline cellulose.

Examples of the disintegrant include
carboxymethylcellulose, carboxymethylcellulose calcium (carmellose calcium), sodium carboxymethyl starch, carmellose sodium, croscarmellose sodium, crospovidone [preferably, Kollidon CL, CL-M, CL-F, CL-SF (trade name, BASF Japan Ltd.); Polyplasdone XL, XL-10, INF-10 (trade name, ISP Japan Co., Ltd.)], low-substituted hydroxypropylcellulose [preferably, low-substituted hydroxypropylcellulose wherein the content of the hydroxypropoxy group is 5 - 16 wt%, such as LH-11, LH-21, LH-31, LH-22, LH-32, LH-20, LH-30, LH-33 (trade name, manufactured by Shin-Etsu Chemical Co., Ltd.) and the like], hydroxypropylstarch, corn starch, and partly pregelatinized starch.

Examples of the binder include hydroxypropylcellulose [preferably, HPC-SSL, SL, L (trade name, NIPPON SODA CO., LTD.)], hydroxypropylmethylcellulose, povidone
(polyvinylpyrrolidone), gum arabic powder, sucrose, gelatin, pullulan, methylcellulose, crystalline cellulose, low-substituted hydroxypropylcellulose [preferably, low-substituted hydroxypropylcellulose wherein the content of the hydroxypropoxy group is 5 - 16 wt%, such as LH-11, LH-21, LH-31, LH-22, LH-32, LH-20, LH-30, LH-33 (trade name, manufactured by Shin-Etsu Chemical Co., Ltd.) and the like], macrogol, dextran, polyvinyl alcohol, and starch glue. Among these, hydroxypropylcellulose is preferable.

Examples of the lubricant include stearic acid, magnesium stearate, calcium stearate, talc, sucrose esters of fatty acids, sodium stearyl fumarate, waxes, DL-leucine, sodium lauryl sulfate, magnesium lauryl sulfate, macrogol, and light anhydrous silicic acid (e.g., AEROSIL). Among these, magnesium stearate is preferable.

Examples of the colorant include food colors such as Food Color Yellow No. 5 (sunset yellow, same as US Food Color yellow No. 6), Food Color Red No. 2, Food Color Blue No. 2 and the like, food lake colors, yellow ferric oxide (yellow ferric oxide), red ferric oxide (red ferric oxide), riboflavin, riboflavin organic acid ester (e.g., riboflavin butyric acid ester), riboflavin phosphate or alkali metal or alkaline earth metal salt thereof, phenolphthalein, titanium oxide, lycopene, and beta-carotene.

Examples of the pH adjuster include citrate, phosphate, carbonate, tartrate, fumarate, acetate, and amino acid salt.

Examples of the surfactant include sodium lauryl sulfate, polysorbate 80, polyoxyethylene(160)polyoxypropylene(30)glycol, polyoxyethylene(196)polyoxypropylene(67)glycol, and polyoxyethylene hydrogenated castor oil 60.

Examples of the stabilizer or antioxidant include sodium ascorbate, tocopherol, tetrasodium edetate, nicotinic acid amide, cyclodextrins; alkaline earth metal salt (e.g., calcium carbonate, calcium hydroxide, magnesium carbonate, magnesium hydroxide, magnesium silicate, magnesium aluminate), and butylhydroxyanisole.

Examples of the corrigent include ascorbic acid, (anhydrous) citric acid, tartaric acid, and malic acid.

Examples of the sweetener include aspartame, acesulfame potassium, thaumatin, saccharin sodium, and dipotassium glycyrrhizinate. Among these, aspartame is preferable.

Examples of the flavor include menthol, peppermint oil, lemon oil, and vanillin.

Examples of the fluidizer include light anhydrous silicic acid and hydrated silicon dioxide. Here, light anhydrous silicic acid may be any as long as it has hydrated silicon dioxide (SiO₂ nH₂O) (n is an integer) as a main component. Specific examples thereof include Sylysia 320 (trade name, Fuji Silysia Chemical Ltd.), AEROSIL 200 (trade name, NIPPON AEROSIL) and the like.

Examples of the antistatic agent include talc and light anhydrous silicic acid.

Examples of the light shielding agent include titanium oxide.

Examples of the antioxidant include dibutylhydroxytoluene (BHT), tocopherol, tocopherol ester (e.g., tocopherol acetate), ascorbic acid or alkali metal or alkaline earth metal salt thereof, lycopene, and beta-carotene.

Examples of the reducing agent include cystine and cysteine.

Examples of the chelating agent include EDTA or alkali metal or alkaline earth metal salt thereof.

Examples of the dosage form of the controlled-release solid preparation of the present invention include tablet, capsule, powder, granule, and troche. Among these, granule, capsule, and tablet are preferable.

The shape of the solid preparation is not particularly limited, and may be any of round, caplet, doughnut, oblong and the like.

The solid preparation and/or granules contained in the solid preparation may be coated with a coating agent as long as it is necessary for achieving the object of the present invention or compatible. In addition, the solid preparation may be stamped with marks and letters for discrimination, and further, a score line for dividing the tablet.

Here, examples of the coating base include sugar coating base, water-soluble film coating base, enteric film coating base, sustained-release film coating base and the like. In addition, the aforementioned enteric polymer, insoluble polymer, and water-soluble polymer may be used.

As the sugar coating base, sucrose is used. Furthermore, one or more kinds selected from talc, precipitated calcium carbonate, gelatin, gum arabic, pullulan, carnauba wax and the like may be used in combination.

Examples of the water-soluble film coating base include water-soluble polymers such as cellulose polymers (hydroxypropylcellulose, hydroxypropylmethylcellulose, hydroxyethylcellulose, methylhydroxyethylcellulose and the like); polyvinyl acetal diethylamino acetate, aminoalkylmethacrylate copolymer E [Eudragit E (trade name) manufactured by EVONIK], polyvinylpyrrolidone; synthetic polymers such as polyvinyl alcohol-polyethylene glycol graft copolymer [Kollicoat IR (trade name) manufactured by BASF] and the like; polysaccharides such as pullulan and the like, and the like.

Examples of the enteric film coating base include cellulose polymers such as hydroxypropylmethylcellulose phthalate, hydroxypropylmethylcellulose acetatesuccinate, carboxymethylethylcellulose, cellulose acetate phthalate and the like; acrylic acid polymers such as methacrylic acid copolymer L [Eudragit L100 (trade name) manufactured by EVONIK], methacrylic acid copolymer LD [Eudragit L-30D55 (trade name) manufactured by EVONIK], methacrylic acid copolymer S [Eudragit S100 (trade name) manufactured by EVONIK] and the like; naturally occurring substances such as shellac and the like, and the like.

Examples of the sustained-release film coating base include insoluble polymers such as cellulose polymers (ethylcellulose, cellulose acetate and the like); acrylic acid polymers (aminoalkylmethacrylate copolymer RS [Eudragit RS (trade name) manufactured by EVONIK], ethyl acrylate-methyl methacrylate copolymer suspension [Eudragit NE (trade name) manufactured by EVONIK] and the like); vinyl polymers (polyvinyl acetate dispersion [Kollicoat SR30D (trade name) manufactured by BASF] and the like) and the like.

Two or more kinds of the above-mentioned coating bases may be mixed and used at an appropriate ratio. In addition, a coating additive may be used in coating.

Examples of the coating additive include light shielding agents and/or colorants such as titanium oxide, talc, red ferric oxide and the like; plasticizers such as polyethylene glycol, triethyl citrate, castor oil, polysorbates and the like, lubricants such as talc and the like, and the like.

The controlled-release solid preparation of the present invention also provides the following effects in addition to the superior effect explained above.

The release rate of the controlled-release solid preparation of the present invention containing a polymer can be controlled by adjusting the amount and molecular weight of the polymer to be used.

The controlled-release solid preparation of the present invention containing immediate-release granule and sustained-release granule; the controlled-release solid preparation of the present invention containing an immediate-release granule and granules coated with a coating layer containing an enteric polymer can control the release rate of a drug by adjusting the contents of these granules.

The controlled-release solid preparation of the present invention containing a water-swellable gel forming polymer (preferably further containing a water-expandable foaming agent component) can control duration in the stomach by adjusting the amount of the polymer (preferably further the foaming agent component) to be used.

While the weight of the controlled-release solid preparation of the present invention is not particularly limited, and varies depending on the dosage form, it is generally 60 - 600 mg, preferably 60 - 480 mg, more preferably 100 - 250 mg.

In the controlled-release solid preparation of the present invention, preparations falling under the following types preferably show the following dissolution pattern.

### Type A

A solid preparation containing pioglitazone or a salt thereof as an active ingredient, constituted of an immediate-release part and a sustained-release part in combination, and having a structure wherein pioglitazone or a salt thereof is contained in both the immediate-release part and the sustained-release part, and the immediate-release part and the sustained-release part are laminated.

### Type B

A solid preparation containing pioglitazone or a salt thereof as an active ingredient, constituted of an immediate-release part and a sustained-release part in combination, and having a structure wherein pioglitazone or a salt thereof is contained in each of the immediate-release part and the sustained-release part, each of the immediate-release part and the sustained-release part has a granule structure, and the sustained-release part is a sustained-release granule comprising an immediate-release granule coated with a coating layer containing a polymer providing sustained release property to the immediate-release granule.

### Type C

A solid preparation containing pioglitazone or a salt thereof as an active ingredient, constituted of an immediate-release part and a sustained-release part in combination, and having a structure wherein pioglitazone or a salt thereof is contained in the immediate-release part, the immediate-release part constitutes an inner core and the other constitutes an outer layer covering the inner core, and the outer layer is a coating layer containing a water-soluble polymer and an insoluble polymer.

### Dissolution pattern [1]

a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.

In the controlled-release solid preparation of the present invention, a preparation falling under the following type preferably shows the following dissolution pattern.

### Type D

A solid preparation containing pioglitazone or a salt thereof as an active ingredient, constituted of an immediate-release part and a sustained-release part in combination, and having a structure wherein pioglitazone or a salt thereof is contained in each of the immediate-release part and the sustained-release part, each of the immediate-release part and the sustained-release part has a granule structure, and the sustained-release part is a sustained-release granule comprising an immediate-release granule coated with a coating layer containing an enteric polymer.

### Dissolution pattern [2]

a dissolution rate of pioglitazone of 10-90% on average at 2 hr time point and 30-110% on average at 4 hr time point, preferably 20-80% on average at 2 hr time point and 40-110% on average at 4 hr time point, in a dissolution test comprising evaluating by the USP basket method at rotation number of 100 rpm, 37°C and using a hydrochloric acid solution (pH 1.2) as a test solution and, 2 hr after dropping the preparation, changing the test solution to a phosphate buffer (pH 6.8)/0.1% CTAB solution and evaluating same under similar conditions.

The controlled-release solid preparation of the present invention can be safely administered orally to mammals (e.g., mouse, rat, rabbit, cat, dog, bovine, horse, monkey, human).

The controlled-release solid preparation of the present invention can be used for the prophylaxis and/or treatment (including delaying onset and suppression of progress) of Alzheimer's disease (particularly, prophylaxis and/or treatment of Alzheimer's dementia).

Alzheimer's disease is one kind of dementia accompanied by change of personality as the main symptom. While there is a theory that Alzheimer's disease is caused by cell degeneration due to intracellular accumulation of β amyloid protein and the like, the theory has not been elucidated. The symptoms progress by steps as shown below along with the atrophy of cerebrum.

### 1) Mild cognitive impairment (sign of Alzheimer)

Mild personality changes such as anxiety, depression, sleep disorder, visual hallucination/delusion and the like appear about 2 - 3 years before decline of intellectual abilities. While mild memory loss is observed, daily living such as calculation of money, driving of car and the like is not hindered.

### 2) Alzheimer first stage

The first stage is also called an amnesia stage, in which amnesia symptom, planotopokinesia, hyperactivity, wandering and the like are observed. As the change of cells, it is the stage when general function of cerebral cortex starts to decline, and starts to exceed the level of simple memory loss.

### 3) Alzheimer second stage

It is also called a confusion stage, atrophy of cerebral cortex progresses to make conversation difficult and the like, and initial symptoms become more serious. High level of mental retardation, aphasia, apraxia and agnosia appear. Extrapyramidal symptoms are sometimes mistaken for those of Parkinson's disease.

### 4) Alzheimer third stage

It is also called a lying in bed stage, and is the late stage of high level of dementia accompanied by being bedridden. Incontinence, refusal to eat, overeating, repetitive motion, spasm and the like occur and aphasia is also observed. Being unable to take care of oneself, caregiving is necessary in all aspects of life.

Since Alzheimer's disease shows gradual progression of symptoms, various biomarkers have been proposed to foresee and prevent the onset in an early stage. The biomarkers include Aβ42 in the cerebrospinal fluid (42 residual fragments of β amyloid protein), tau protein in the cerebrospinal fluid, apolipoprotein E (ApoE) ε4 allele number, and the like. On the other hand, TOMM40 (mitochondrial outer membrane channel subunit, 40 kDa) has been reported to specifically interact with ApoE colocalized in the mitochondrial outer membrane to induce mitochondrial apoptosis (PCT/US 2009/053373).

The controlled-release solid preparation of the present invention is desirably administered to patients with a high risk of developing Alzheimer's disease by these biomarkers to prevent the onset thereof.

The "treatment of Alzheimer's dementia" in the present specification includes various embodiments below.
1) delaying the onset of mild cognitive impairment caused by Alzheimer's disease
2) suppression of progress of cognitive decline in the subject who has developed mild cognitive impairment caused by Alzheimer's disease
3) suppression of progress of cognitive impairment in the subject who has developed mild cognitive impairment caused by Alzheimer's disease
4) prevention of onset of Alzheimer's disease in the subject who has developed mild cognitive impairment caused by Alzheimer's disease, and
5) improvement of cognitive impairment in the subject who has developed mild cognitive impairment caused by Alzheimer's disease.

The controlled-release solid preparation of the present invention can be used in combination with an active ingredient other than pioglitazone or a salt thereof (hereinafter sometimes to be abbreviated as combined use component). In this case, the administration periods of pioglitazone or a salt thereof and the combined use component are not limited, and they may be administered simultaneously to the administration subject or may be administered in a staggered manner. Furthermore, the controlled-release solid preparation of the present invention and the combined use component may be administered as two kinds of preparations containing each active ingredient, or may be administered as a single preparation containing the both active ingredients.

The dose of the combined use component can be appropriately determined based on a clinically-employed dose as the standard.

Using a combined use component, superior effects can be obtained such as 1) enhancing effect on the action of the controlled-release solid preparation of the present invention or a combined use component (synergistic effect of drug action), 2) reducing effect on the dose of the controlled-release solid preparation of the present invention or a combined use component (reducing effect on the dose of a drug as compared to single administration), 3) reducing effect on the secondary action of the controlled-release solid preparation of the present invention or a combined use component and the like.

Examples of the combined use component include other drugs useful as prophylactic and/or therapeutic agents for Alzheimer's disease.

The present invention also relates to a method for the prophylaxis and/or treatment of Alzheimer's dementia, comprising administering the above-mentioned controlled-release solid preparation of the present invention to a subject in need of the administration.

The dose, administration method and the like are the same as the amount, method and the like explained for the above-mentioned controlled-release solid preparation of the present invention.

### Examples

The present invention is explained in more detail in the following by referring to Examples and Experimental Examples, which are not to be construed as limitative.

### Examples 1 - 5 Matrix two-layer tablet (IR layer and SR layer)

According to the formulations of Tables 1-1 - 1-5, pioglitazone hydrochloride (fine pulverized product, average particle size about 5 µm; measured value by Mastersizer 2000 (hereinafter the same in the Examples)), crystalline cellulose, lactose and carmellose calcium were uniformly mixed in a mortar, and magnesium stearate was added. The mixture was further uniformly mixed and granules for tableting were obtained by the wet granulation method (IR layer). Similarly, according to the formulations of Tables 1-1 - 1-5, pioglitazone hydrochloride (fine pulverized product, average particle size about 5 µm), crystalline cellulose, D-mannitol and polyethylene oxide (Polyox various viscosity grades, manufactured by Dow) were uniformly mixed, butylhydroxyanisole and sodium stearyl fumarate were added, and the mixture was further uniformly mixed to give granules for tableting (SR layer). These were each measured by 120 mg at the ratio of Table 1, and the IR layer was tableted by a hand presser (HAND TAB-200, ICHIHASHISEIKI) using a 7 mmϕ punch. The granules of the SR layer were further added and tableted to give 20 core tablets containing 1 mg of pioglitazone per tablet.

**Table 1**

| | IR/SR drug substance ratio | IR layer drug substance amount | SR layer drug substance amount |
|---|---|---|---|
| Example 1 | IR:SR=2:1 | 0.67 mg | 0.33 mg |
| Example 2 | IR:SR=1:2 | 0.33 mg | 0.67 mg |
| Example 3 | IR:SR=5:1 | 0.83 mg | 0.17 mg |
| Example 4 | IR:SR=1:5 | 0.17 mg | 0.83 mg |
| Example 5 | IR:SR=1:9 | 0.1 mg | 0.9 mg |

**[Table 1-1]**

| Example 1 | | |
|---|---|---|
| additive | function | formulation amount (mg/tablet) |
| IR layer | | |
| pioglitazone hydrochloride | active ingredient | 0.74 |
| (pioglitazone free form) | | (0.67) |
| crystalline cellulose | excipient | 0.2 |
| lactose | excipient | 111.46 |
| carmellose calcium | disintegrant | 7.2 |
| magnesium stearate | lubricant | 0.4 |
| Total | | 120 |

| SR layer | | |
|---|---|---|
| pioglitazone hydrochloride | active ingredient | 0.36 |
| (pioglitazone free form) | | (0.33) |
| crystalline cellulose | gel forming promoter | 0.1 |
| D-mannitol | gel forming promoter | 82.22 |
| polyethylene oxide (Polyox N-12K) | gel forming polymer | 36 |
| butylhydroxyanisole | antioxidant | 0.12 |
| sodium stearyl fumarate | lubricant | 1.2 |
| Total | | 120 |

**[Table 1-2]**

| Example 2 | | |
|---|---|---|
| additive | function | formulation amount (mg/tablet) |
| IR layer | | |
| pioglitazone hydrochloride | active ingredient | 0.36 |
| (pioglitazone free form) | | (0.33) |
| crystalline cellulose | excipient | 0.1 |
| lactose | excipient | 111.94 |
| carmellose calcium | disintegrant | 7.2 |
| magnesium stearate | lubricant | 0.4 |
| Total | | 120 |

| SR layer | | |
|---|---|---|
| pioglitazone hydrochloride | active ingredient | 0.74 |
| (pioglitazone free form) | | (0.67) |
| crystalline cellulose | gel forming promoter | 0.2 |
| D-mannitol | gel forming promoter | 81.74 |
| polyethylene oxide (Polyox N-12K) | gel forming polymer | 36 |
| butylhydroxyanisole | antioxidant | 0.12 |
| sodium stearyl fumarate | lubricant | 1.2 |
| Total | | 120 |

**[Table 1-3]**

| Example 3 | | |
|---|---|---|
| additive | function | formulation amount (mg/tablet) |
| IR layer | | |
| pioglitazone hydrochloride | active ingredient | 0.92 |
| (pioglitazone free form) | | (0.83) |
| crystalline cellulose | excipient | 0.25 |
| lactose | excipient | 111.23 |
| carmellose calcium | disintegrant | 7.2 |
| magnesium stearate | lubricant | 0.4 |
| Total | | 120 |

| SR layer | | |
|---|---|---|
| pioglitazone hydrochloride | active ingredient | 0.18 |
| (pioglitazone free form) | | (0.17) |
| crystalline cellulose | gel forming promoter | 0.05 |
| D-mannitol | gel forming promoter | 82.44 |
| polyethylene oxide (Polyox N-12K) | gel forming polymer | 36 |
| butylhydroxyanisole | antioxidant | 0.12 |
| sodium stearyl fumarate | lubricant | 1.2 |
| Total | | 120 |

**[Table 1-4]**

| Example 4 | | |
|---|---|---|
| additive | function | formulation amount (mg/tablet) |
| IR layer | | |
| pioglitazone hydrochloride | active ingredient | 0.18 |
| (pioglitazone free form) | | (0.17) |
| crystalline cellulose | excipient | 0.05 |
| lactose | excipient | 112.16 |
| carmellose calcium | disintegrant | 7.2 |
| magnesium stearate | lubricant | 0.4 |
| Total | | 120 |

| SR layer | | |
|---|---|---|
| pioglitazone hydrochloride | active ingredient | 0.92 |
| (pioglitazone free form) | | (0.83) |
| crystalline cellulose | gel forming promoter | 0.25 |
| D-mannitol | gel forming promoter | 81.51 |
| polyethylene oxide (Polyox N-12K) | gel forming polymer | 36 |
| butylhydroxyanisole | antioxidant | 0.12 |
| sodium stearyl fumarate | lubricant | 1.2 |
| Total | | 120 |

**[Table 1-5]**

| Example 5 | | |
|---|---|---|
| additive | function | formulation amount (mg/tablet) |
| IR layer | | |
| pioglitazone hydrochloride | active ingredient | 0.11 |
| (pioglitazone free form) | | (0.1) |
| crystalline cellulose | excipient | 0.03 |
| lactose | excipient | 112.26 |
| carmellose calcium | disintegrant | 7.2 |
| magnesium stearate | lubricant | 0.4 |
| Total | | 120 |

| SR layer | | |
|---|---|---|
| pioglitazone hydrochloride | active ingredient | 0.99 |
| (pioglitazone free form) | | (0.9) |
| crystalline cellulose | gel forming promoter | 0.27 |
| D-mannitol | gel forming promoter | 81.44 |
| polyethylene oxide (Polyox N-12K) | gel forming polymer | 36 |
| butylhydroxyanisole | antioxidant | 0.12 |
| sodium stearyl fumarate | lubricant | 1.2 |
| Total | | 120 |

### Examples 6 - 10 Membrane-controlled granule preparation (capsule preparation filled with IR granule and DR granule)

Using a vertical granulator (FM-VG-10, POWREX CORPORATION) and according to the formulations of Tables 2-1 - 2-3, pioglitazone hydrochloride (fine pulverized product, average particle size about 5 µm), crystalline cellulose, corn starch, low-substituted hydroxypropylcellulose and sucrose were uniformly mixed to give a spray powder. In CF apparatus (CF-260, FREUND), the spray powder and hydroxypropylcellulose were sprayed on Nonpareil, and they were dried by a vacuum dryer to give IR granules. Separately, coating solutions composed of Eudragit (manufactured by EVONIK), triethyl citrate and talc according to the formulations of Tables 2-1 - 2-3, were applied to IR granule by a fluid bed granulator (FD-3S, POWREX CORPORATION), and dried therein to give DR granule. The IR granule and DR granule were respectively filled in a capsule at the ratio shown in Table 2 to give a capsule containing 1 mg of pioglitazone per one capsule.

**Table 2**

| | IR:DR drug substance ratio | IR granule drug substance amount | DR granule drug substance amount |
|---|---|---|---|
| Example 6 | IR:DR=2:1, pH 5.5 | 0.67 mg | 0.33 mg |
| Example 7 | IR:DR=2:1, pH 6.5 | 0.67 mg | 0.33 mg |
| Example 8 | IR:DR=1:1, pH 5.5 | 0.5 mg | 0.5 mg |
| Example 9 | IR:DR=1:1, pH 6.0 | 0.5 mg | 0.5 mg |
| Example 10 | IR:DR=1:1, pH 6.5 | 0.5 mg | 0.5 mg |

**[Table 2-1]**

| Examples 6, 8 | | |
|---|---|---|
| additive | function | formulation amount (mg/tablet) |
| IR granule | | |
| pioglitazone hydrochloride | active ingredient | 1.1 |
| (pioglitazone free form) | | (1) |
| crystalline cellulose | excipient | 0.28 |
| corn starch | excipient | 15.82 |
| low-substituted hydroxypropylcellulose | binder | 12.8 |
| sucrose | excipient | 25.6 |
| hydroxypropylcellulose | binder | 0.4 |
| Nonpareil (101-750) | core granule | 24 |
| Total | | 80 |

| DR coating (pH 5.5) | | |
|---|---|---|
| Eudragit L30D-55 | enteric polymer | 40 (solid content 12) |
| triethyl citrate | plasticizer | 1.2 |
| talc | lubricant | 3.6 |
| Total | | 16.8 (solid content) |

**[Table 2-2]**

| Examples 7, 10 | | |
|---|---|---|
| additive | function | formulation amount (mg/tablet) |
| IR granule | | |
| pioglitazone hydrochloride | active | 1.1 |
| (pioglitazone free form) | ingredient | (1) |
| crystalline cellulose | excipient | 0.28 |
| corn starch | excipient | 15.82 |
| low-substituted hydroxypropylcellulose | binder | 12.8 |
| sucrose | excipient | 25.6 |
| hydroxypropylcellulose | binder | 0.4 |
| Nonpareil (101-750) | core granule | 24 |
| Total | | 80 |

| DR coating (pH 6.5) | | |
|---|---|---|
| Eudragit L100 | enteric polymer | 8 |
| Eudragit S100 | enteric polymer | 8 |
| triethyl citrate | plasticizer | 1.6 |
| talc | lubricant | 8 |
| Total | | 25.6 (solid content) |

**[Table 2-3]**

| Example 9 | | |
|---|---|---|
| additive | function | formulation amount (mg/tablet) |
| IR granule | | |
| pioglitazone hydrochloride | active ingredient | 1.1 |
| (pioglitazone free form) | | (1) |
| crystalline cellulose | excipient | 0.28 |
| corn starch | excipient | 15.82 |
| low-substituted hydroxypropylcellulose | binder | 12.8 |
| sucrose | excipient | 25.6 |
| hydroxypropylcellulose | binder | 0.4 |
| Nonpareil (101-750) | core granule | 24 |
| Total | | 80 |

| DR coating (pH 6.0) | | |
|---|---|---|
| Eudragit L100 | enteric polymer | 24 |
| triethyl citrate | plasticizer | 12 |
| talc | lubricant | 12 |
| Total | | 48 (solid content) |

### Examples 11 - 14 Membrane-controlled granule preparation (capsule preparation filled with SR granule)

Using a vertical granulator (FM-VG-10, POWREX CORPORATION) and according to the formulations of Tables 3-1 - 3-4, pioglitazone hydrochloride (fine pulverized product, average particle size about 5 µm), crystalline cellulose, corn starch, low-substituted hydroxypropylcellulose and sucrose were uniformly mixed to give a spray powder. In CF apparatus (CF-260, FREUND), the spray powder and hydroxypropylcellulose were sprayed on Nonpareil, and they were dried by a vacuum dryer to give IR granules. Separately, coating solutions composed of Kollicoat SR 30D (manufactured by BASF), Kollicoat IR (manufactured by BASF), triethyl citrate and talc according to the formulations of Tables 3-1 - 3-4, were applied to IR granule by a fluid bed granulator (FD-3S, POWREX CORPORATION) to give SR granule. The SR granule was weighted and filled in a capsule to give a preparation to contain 1 mg of pioglitazone per one capsule.

**[Table 3-1]**

| Example 11 | | |
|---|---|---|
| additive | function | formulation amount (mg/tablet) |
| IR granule | | |
| pioglitazone hydrochloride | active ingredient | 1.1 |
| (pioglitazone free form) | | (1) |
| crystalline cellulose | excipient | 0.28 |
| corn starch | excipient | 15.82 |
| low-substituted hydroxypropylcellulose | binder | 12.8 |
| sucrose | excipient | 25.6 |
| hydroxypropylcellulose | binder | 0.4 |
| Nonpareil (101-750) | core granule | 24 |
| Total | | 80 |

| SR coating | | |
|---|---|---|
| Kollicoat SR 30D | insoluble polymer | 8 |
| Kollicoat IR | water-soluble polymer | 3.4 |
| triethyl citrate | plasticizer | 0.58 |
| talc | lubricant | 4.58 |
| Total | | 16.56 (solid content) |

**[Table 3-2]**

| Example 12 | | |
|---|---|---|
| additive | function | formulation amount (mg/tablet) |
| IR granule | | |
| pioglitazone hydrochloride | active ingredient | 1.1 |
| (pioglitazone free form) | | (1) |
| crystalline cellulose | excipient | 0.28 |
| corn starch | excipient | 15.82 |
| low-substituted hydroxypropylcellulose | binder | 12.8 |
| sucrose | excipient | 25.6 |
| hydroxypropylcellulose | binder | 0.4 |
| Nonpareil (101-750) | core granule | 24 |
| Total | | 80 |

| SR coating | | |
|---|---|---|
| Kollicoat SR 30D | insoluble polymer | 12 |
| Kollicoat IR | water-soluble polymer | 5.1 |
| triethyl citrate | plasticizer | 0.87 |
| talc | lubricant | 6.87 |
| Total | | 24.84 (solid content) |

**[Table 3-3]**

| Example 13 | | |
|---|---|---|
| additive | function | formulation amount (mg/tablet) |
| IR granule | | |
| pioglitazone hydrochloride | active ingredient | 1.1 |
| (pioglitazone free form) | | (1) |
| crystalline cellulose | excipient | 0.28 |
| corn starch | excipient | 15.82 |
| low-substituted hydroxypropylcellulose | binder | 12.8 |
| sucrose | excipient | 25.6 |
| hydroxypropylcellulose | binder | 0.4 |
| Nonpareil (101-750) | core granule | 24 |
| Total | | 80 |

| SR coating | | |
|---|---|---|
| Kollicoat SR 30D | insoluble polymer | 8 |
| Kollicoat IR | water-soluble polymer | 2 |
| triethyl citrate | plasticizer | 0.5 |
| talc | lubricant | 4 |
| Total | | 14.5 (solid content) |

**[Table 3-4]**

| Example 14 | | |
|---|---|---|
| additive | function | formulation amount (mg/tablet) |
| IR granule | | |
| pioglitazone hydrochloride | active ingredient | 1.1 |
| (pioglitazone free form) | | (1) |
| crystalline cellulose | excipient | 0.28 |
| corn starch | excipient | 15.82 |
| low-substituted hydroxypropylcellulose | binder | 12.8 |
| sucrose | excipient | 25.6 |
| hydroxypropylcellulose | binder | 0.4 |
| Nonpareil (101-750) | core granule | 24 |
| Total | | 80 |

| SR coating | | |
|---|---|---|
| Kollicoat SR 30D | insoluble polymer | 12 |
| Kollicoat IR | water-soluble polymer | 3 |
| triethyl citrate | plasticizer | 0.75 |
| talc | lubricant | 6 |
| Total | | 21.75 (solid content) |

### Example 15 Membrane-controlled sustained-release preparation (IR tablet and SR coating)

According to the formulation of Table 4-1, pioglitazone hydrochloride (fine pulverized product, average particle size about 5 µm), crystalline cellulose, lactose and corn starch were uniformly mixed in a fluid bed dryer granulator (MP-01, POWREX CORPORATION), a dispersion of hydroxypropylcellulose was sprayed thereon for granulation, and the granules were dried therein. The obtained granules were sieved with a 16M sieve to give a sieved granules. Magnesium stearate and corn starch were added to the sieved granules, and they were mixed by hand in a 10L plastic bag to give granules for tableting. The granules were measured by 120 mg, and tableted by a compact tableting machine (VEL5, Kikusui Seisakusho Ltd.) using a 7 mmϕ punch to 120 mg weight to give 5,000 core tablets containing 1 mg of pioglitazone per tablet. The obtained tablets were film coated according to Table 4-2 by a dria coater (DRC-200, POWREX CORPORATION) to give film-coated tablets.

**[Table 4-1]**

| IR tablet | | |
|---|---|---|
| additive | function | formulation amount (mg/tablet) |
| pioglitazone hydrochloride | active ingredient | 1.1 |
| (pioglitazone free form) | | (1) |
| crystalline cellulose | excipient | 0.28 |
| lactose | excipient | 77.16 |
| corn starch | disintegrant | 37.26 |
| hydroxypropylcellulose | binder | 3.6 |
| magnesium stearate | lubricant | 0.6 |
| Total | | 120 |

**[Table 4-2]**

| SR coating (polymer 10% coating) | | |
|---|---|---|
| additive | function | formulation amount (mg/tablet) |
| Kollicoat SR 30D | insoluble polymer | 6 |
| Kollicoat IR | water-soluble polymer | 6 |
| triethyl citrate | plasticizer | 0.6 |
| talc | lubricant | 4.8 |
| Total | | 17.4 |

### Examples 16 - 18 Intragastric floating preparation (foaming component and highly swellable polymer)

According to the formulations of Table 5-1 - 5-3, pioglitazone hydrochloride (fine pulverized product, average particle size about 5 µm), crystalline cellulose, D-mannitol, polyethylene oxide (Polyox various viscosity grades, manufactured by Dow), citric acid and sodium hydrogen carbonate were uniformly mixed in a mortar, sodium stearyl fumarate was added, and the mixture was further mixed uniformly to give granules for tableting. The granules were measured by 120 mg, and tableted by Autograph (AG-1 type, Shimadzu Corporation) using a 7 mmϕ punch to give 10 core tablets containing 1 mg of pioglitazone per tablet.

**[Table 5-1]**

| Example 16 | | |
|---|---|---|
| additive | function | formulation amount (mg/tablet) |
| pioglitazone hydrochloride | active ingredient | 1.1 |
| (pioglitazone free form) | | (1) |
| crystalline cellulose | gel forming promoter | 0.28 |
| D-mannitol | gel forming promoter | 45.42 |
| polyethylene oxide (Polyox N-12K) | gel forming polymer | 36 |
| citric acid | foaming agent | 15.5 |
| sodium hydrogen carbonate | foaming agent | 20.5 |
| sodium stearyl fumarate | lubricant | 1.2 |
| Total | | 120 |

**[Table 5-2]**

| Example 17 | | |
|---|---|---|
| additive | function | formulation amount (mg/tablet) |
| pioglitazone hydrochloride | active ingredient | 1.1 |
| (pioglitazone free form) | | (1) |
| crystalline cellulose | gel forming promoter | 0.28 |
| D-mannitol | gel forming promoter | 33.42 |
| polyethylene oxide (Polyox N-12K) | gel forming polymer | 48 |
| citric acid | foaming agent | 15.5 |
| sodium hydrogen carbonate | foaming agent | 20.5 |
| sodium stearyl fumarate | lubricant | 1.2 |
| Total | | 120 |

**[Table 5-3]**

| Example 18 | | |
|---|---|---|
| additive | function | formulation amount (mg/tablet) |
| pioglitazone hydrochloride | active ingredient | 1.1 |
| (pioglitazone free form) | | (1) |
| crystalline cellulose | gel forming promoter | 0.28 |
| D-mannitol | gel forming promoter | 21.42 |
| polyethylene oxide (Polyox N-12K) | gel forming polymer | 60 |
| citric acid | foaming agent | 15.5 |
| sodium hydrogen carbonate | foaming agent | 20.5 |
| sodium stearyl fumarate | lubricant | 1.2 |
| Total | | 120 |

### Examples 19 - 20 Nucleated tablet (IR layer and SR layer)

According to the formulations of Tables 6-1 - 6-2, pioglitazone hydrochloride (fine pulverized product, average particle size about 5 µm), crystalline cellulose, lactose and carmellose calcium were uniformly mixed in a mortar, and magnesium stearate was added. The mixture was further uniformly mixed and granules for tableting were obtained by the wet granulation method (IR layer). Similarly, according to the formulations of Tables 6-1 - 6-2, pioglitazone hydrochloride (fine pulverized product, average particle size about 5 µm), crystalline cellulose, D-mannitol and polyethylene oxide (Polyox various viscosity grades, manufactured by Dow) were uniformly mixed, butylhydroxyanisole and sodium stearyl fumarate were added, and the mixture was further uniformly mixed to give granules for tableting (SR layer). The granules were measured by 120 mg for the SR layer and by 300 mg for the IR layer to meet the ratios in Table 6. The SR layer was tableted by a hand presser (HAND TAB-200, ICHIHASHISEIKI) using a 7 mmϕ punch, and then the IR layer (150 mg) was weakly tableted using a 10 mmϕ punch. The SR tablet was placed to be the center, the remaining IR layer (150 mg) was added and the mixture was tableted to give 20 core tablets containing 1 mg of pioglitazone per tablet.

**[Table 6]**

| | IR/SR drug substance ratio | IR layer drug substance amount | SR layer drug substance amount |
|---|---|---|---|
| Example 19 | IR:SR=2:1 | 0.67 mg | 0.33 mg |
| Example 20 | IR:SR=1:2 | 0.33 mg | 0.67 mg |

**[Table 6-1]**

| Example 19 | | |
|---|---|---|
| additive | function | formulation amount (mg/tablet) |
| IR layer | | |
| pioglitazone hydrochloride | active ingredient | 0.74 |
| (pioglitazone free form) | | (0.67) |
| crystalline cellulose | excipient | 0.2 |
| lactose | excipient | 280.06 |
| carmellose calcium | disintegrant | 18 |
| magnesium stearate | lubricant | 1 |
| Total | | 300 |

| SR layer | | |
|---|---|---|
| pioglitazone hydrochloride | active ingredient | 0.36 |
| (pioglitazone free form) | | (0.33) |
| crystalline cellulose | gel forming promoter | 0.10 |
| D-mannitol | gel forming promoter | 82.22 |
| polyethylene oxide | gel forming polymer | 36 |
| butylhydroxyanisole | antioxidant | 0.12 |
| sodium stearyl fumarate | lubricant | 1.2 |
| Total | | 120 |

**[Table 6-2]**

| Example 20 | | |
|---|---|---|
| additive | function | formulation amount (mg/tablet) |
| IR layer | | |
| pioglitazone hydrochloride | active ingredient | 0.36 |
| (pioglitazone free form) | | (0.33) |
| crystalline cellulose | excipient | 0.1 |
| lactose | excipient | 280.54 |
| carmellose calcium | disintegrant | 18 |
| magnesium stearate | lubricant | 1 |
| Total | | 300 |

| SR layer | | |
|---|---|---|
| pioglitazone hydrochloride | active ingredient | 0.74 |
| (pioglitazone free form) | | (0.67) |
| crystalline cellulose | gel forming promoter | 0.2 |
| D-mannitol | gel forming promoter | 81.74 |
| polyethylene oxide | gel forming polymer | 36 |
| butylhydroxyanisole | antioxidant | 0.12 |
| sodium stearyl fumarate | lubricant | 1.2 |
| Total | | 120 |

### Example 21 (OROS type preparation)

According to the formulation of Table 7, pioglitazone hydrochloride (fine pulverized product, average particle size about 5 µm) and polyethylene oxide are uniformly mixed in a mortar, and magnesium stearate is added. The mixture is further mixed uniformly to give granules for tableting (drug layer). Similarly, according to the formulation of Table 7, D-mannitol and polyethylene oxide (Polyox various viscosity grades, manufactured by Dow) are uniformly mixed, sodium stearyl fumarate is added, and the mixture is further mixed uniformly to give granules for tableting (PUSH layer). They are measured by 100 mg each, and the drug layer is tableted by a hand presser (HAND TAB-200, ICHIHASHISEIKI) using a 7 mmϕ punch. The granules of the PUSH layer are further added and the mixture is tableted to give core tablets containing 1 mg of pioglitazone per tablet. The core tablets are filled in capsules by one tablet each, the capsules are coated with a solution of Opadry CA in acetone-water mixed solution (solid content 7%, acetone-water mixed solution (90:10)) and dried. Using a 25G injection needle, a hole is made in the upper part of the capsule on the drug layer side to give a preparation.

**[Table 7]**

| Example 21 | | |
|---|---|---|
| additive | function | formulation amount (mg/tablet) |
| drug layer | | |
| pioglitazone hydrochloride | active ingredient | 1.1 |
| (pioglitazone free form) | | (1) |
| polyethylene oxide (Polyox N-80) | gel forming polymer | 98.6 |
| magnesium stearate | lubricant | 0.3 |
| Total | | 100 |

| Push layer | | |
|---|---|---|
| polyethylene oxide (Polyox Coagulant) | gel forming polymer | 64 |
| D-mannitol | gel forming promoter | 35 |
| sodium stearyl fumarate | lubricant | 1 |
| Total | | 100 |

### Experimental Example 1 (dissolution test)

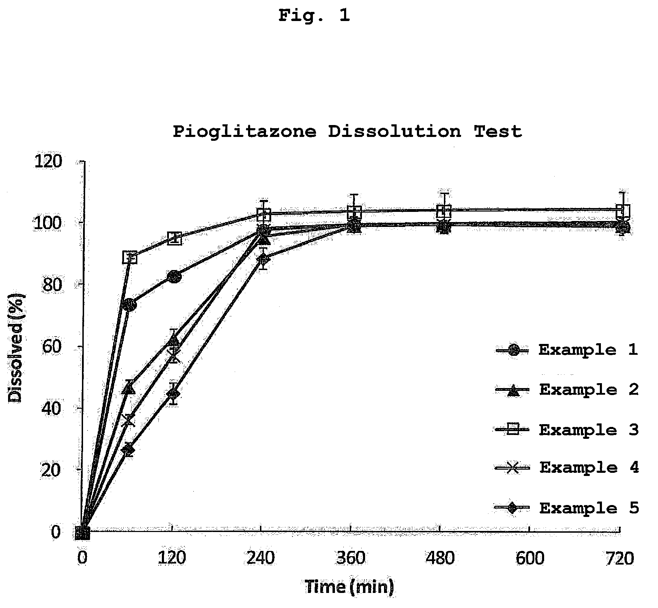

The tablets obtained in Examples 1 - 5 were measured for dissolution property. One tablet was dropped in 900 mL of 0.3M potassium chloride buffer (pH 2), and evaluated by the USP paddle method at rotation number of 50 rpm, 37°C. After dropping the tablet, the test solution was sampled over time, filtered through a non-aqueous filter (0.45 µm), and quantified by the high-speed liquid column chromatography (HPLC) method under the following conditions, and the dissolution rate was calculated. The results are shown in Fig. 1 and Table 8.

### <HPLC conditions>

detector: UV absorption spectrophotometer, measurement
wavelength: 269 nm
column: YMC-Pack ODS-A A-302, 5 µm, inner diameter: 4.6 mm,
length: 75 mm
column temperature: 25°C
mobile phase: acetonitrile:0.1 mol/L ammonium acetate
buffer:glacial acetic acid mixed solution (25:25:1)
flow: 0.7 mL/min

**[Table 8]**

| Time (min) | 0 | 60 | 120 | 240 | 360 | 480 | 720 |
|---|---|---|---|---|---|---|---|
| Example 1 | 0 | 74 | 83 | 98 | 100 | 99 | 99 |
| Example 2 | 0 | 47 | 63 | 95 | 99 | 100 | 99 |
| Example 3 | 0 | 89 | 95 | 103 | 104 | 104 | 104 |
| Example 4 | 0 | 36 | 57 | 98 | 99 | 100 | 100 |
| Example 5 | 0 | 27 | 45 | 89 | 99 | 99 | 99 |

### Experimental Example 2 (acid resistance test/dissolution test)

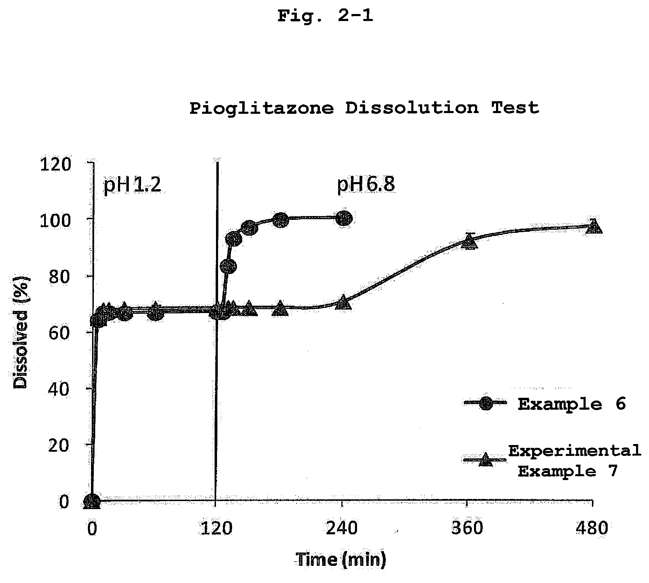

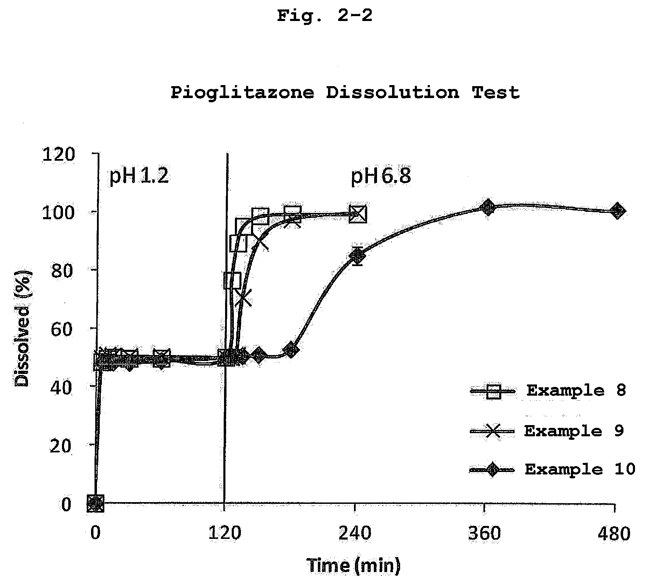

The capsules obtained in Examples 6 - 10 were measured for acid resistance and dissolution property. One capsule was dropped in 900 mL of hydrochloric acid solution (pH 1.2), and evaluated by the USP basket method at rotation number of 100 rpm, 37°C. At 2 hr after dropping the capsule, the test solution was changed to 900 mL of phosphate buffer (pH 6.8)/0.1% CTAB solution, and the capsule was evaluated under similar conditions. After dropping the capsule, the test solution was sampled over time, filtered through a non-aqueous filter (0.45 µm), and quantified by the high-speed liquid column chromatography (HPLC) method under the following conditions, and the dissolution rate was calculated. The results are shown in Figs. 2-1 - 2-2 and Table 9.

### <HPLC conditions>

detector: UV absorption spectrophotometer, measurement
wavelength: 269 nm
column: YMC-Pack ODS-A A-302, 5 µm, inner diameter: 4.6 mm,
length: 75 mm
column temperature: 25°C
mobile phase: acetonitrile:0.1 mol/L ammonium acetate
buffer:glacial acetic acid mixed solution (25:25:1)
flow: 0.7 mL/min

**[Table 9]**

| Time (min) | 0 | 5 | 10 | 15 | 30 | 60 | 120 | 125 | 130 | 135 | 150 | 180 | 240 | 360 | 480 |
|---|---|---|---|---|---|---|---|---|---|---|---|---|---|---|---|
| Example 6 | 0 | 65 | 68 | 68 | 68 | 68 | 68 | 84 | 93 | 97 | 99 | 100 | 100 | - | - |
| Example 7 | 0 | 65 | 68 | 68 | 68 | 68 | 68 | 69 | 69 | 69 | 69 | 69 | 71 | 92 | 98 |
| Example 8 | 0 | 48 | 49 | 50 | 50 | 50 | 50 | 76 | 89 | 95 | 98 | 99 | 99 | - | - |
| Example 9 | 0 | 50 | 51 | 51 | 50 | 50 | 50 | 50 | 51 | 71 | 90 | 97 | 100 | - | - |
| Example 10 | 0 | 48 | 48 | 48 | 48 | 49 | 50 | 50 | 50 | 51 | 51 | 53 | 85 | 102 | 101 |

### Experimental Example 3 (dissolution test)

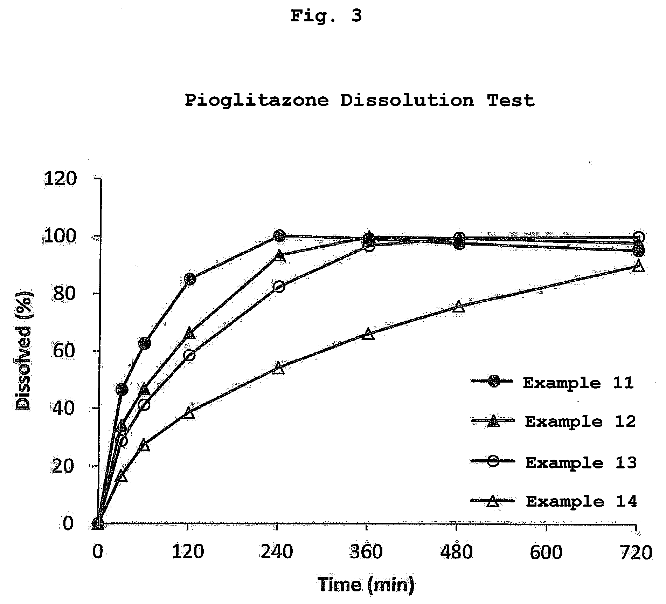

The capsules obtained in Examples 11 - 14 were measured for dissolution property. One capsule was dropped in 900 mL of 0.3M potassium chloride buffer (pH 2), and evaluated by the USP basket method at rotation number of 100 rpm, 37°C. After dropping the capsule, the test solution was sampled over time, filtered through a non-aqueous filter (0.45 µm), and quantified by the high-speed liquid column chromatography (HPLC) method under the following conditions, and the dissolution rate was calculated. The results are shown in Fig. 3 and Table 10.

### <HPLC conditions>

detector: UV absorption spectrophotometer, measurement
wavelength: 269 nm
column: YMC-Pack ODS-A A-302, 5 µm, inner diameter: 4.6 mm,
length: 75 mm
column temperature: 25°C
mobile phase: acetonitrile:0.1 mol/L ammonium acetate
buffer:glacial acetic acid mixed solution (25:25:1)
flow: 0.7 mL/min

**[Table 10]**

| Time (min) | 0 | 30 | 60 | 120 | 240 | 360 | 480 | 720 |
|---|---|---|---|---|---|---|---|---|
| Example 11 | 0 | 47 | 63 | 85 | 100 | 99 | 98 | 95 |
| Example 12 | 0 | 34 | 47 | 66 | 93 | 100 | 99 | 98 |
| Example 13 | 0 | 29 | 41 | 59 | 82 | 97 | 99 | 100 |
| Example 14 | 0 | 17 | 27 | 39 | 54 | 66 | 76 | 90 |

### Experimental Example 4 (dissolution test)

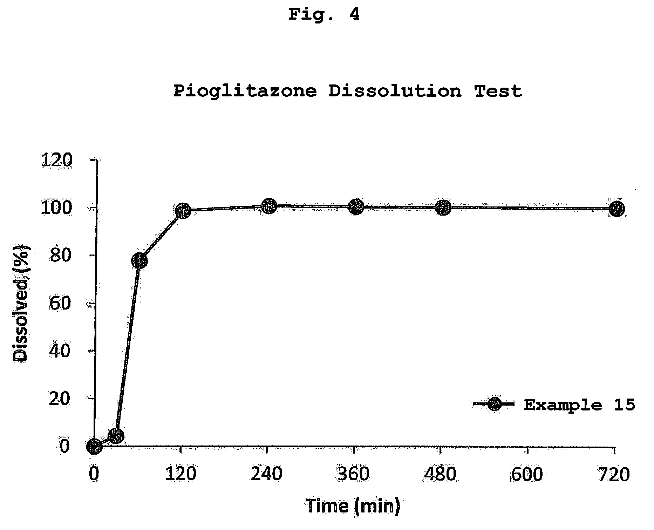

The tablet obtained in Example 15 was measured for dissolution property. One tablet was dropped in 900 mL of 0.3M potassium chloride buffer (pH 2), and evaluated by the USP paddle method at rotation number of 50 rpm, 37°C. After dropping the tablet, the test solution was sampled over time, filtered through a non-aqueous filter (0.45 µm), and quantified by the high-speed liquid column chromatography (HPLC) method under the following conditions, and the dissolution rate was calculated. The results are shown in Fig. 4 and Table 11.

### <HPLC conditions>

detector: UV absorption spectrophotometer, measurement
wavelength: 269 nm
column: YMC-Pack ODS-A A-302, 5 µm, inner diameter: 4.6 mm,
length: 75 mm
column temperature: 25°C
mobile phase: acetonitrile:0.1 mol/L ammonium acetate
buffer:glacial acetic acid mixed solution (25:25:1)
flow: 0.7 mL/min

**[Table 11]**

| Time (min) | 0 | 30 | 60 | 120 | 240 | 360 | 480 | 720 |
|---|---|---|---|---|---|---|---|---|
| Example 15 | 0 | 4.4 | 78 | 99 | 101 | 101 | 100 | 100 |

### Experimental Example 5 (dissolution test)

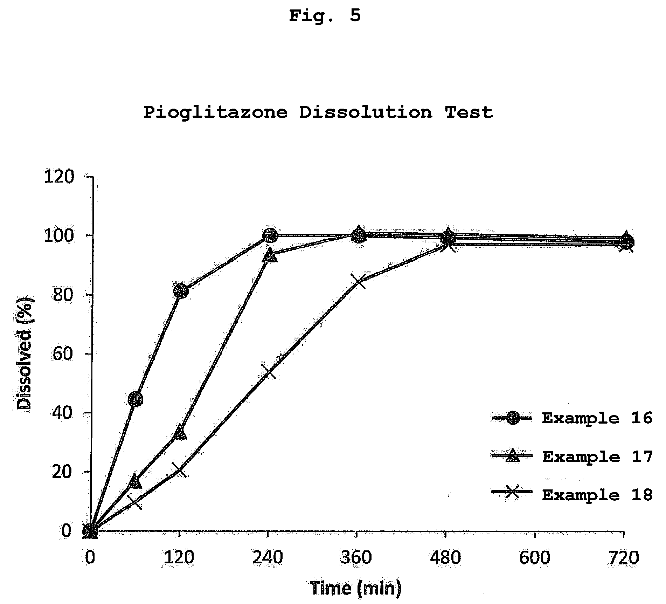

The tablets obtained in Examples 16 - 18 were measured for dissolution property. One tablet was dropped in 900 mL of 0.3M potassium chloride buffer (pH 2), and evaluated by the USP paddle method at a rotation number 50 rpm, 37°C. After dropping the tablet, the test solution was sampled over time, filtered through a non-aqueous filter (0.45 µm), and quantified by the high-speed liquid column chromatography (HPLC) method under the following conditions, and the dissolution rate was calculated. The results are shown in Fig. 5 and Table 12.

### <HPLC conditions>

detector: UV absorption spectrophotometer, measurement
wavelength: 269 nm
column: YMC-Pack ODS-A A-302, 5 µm, inner diameter: 4.6 mm,
length:75 mm
column temperature: 25°C
mobile phase: acetonitrile:0.1 mol/L ammonium acetate
buffer:glacial acetic acid mixed solution (25:25:1)
flow: 0.7 mL/min

**[Table 12]**

| Time (min) | 0 | 60 | 120 | 240 | 360 | 480 | 720 |
|---|---|---|---|---|---|---|---|
| Example 16 | 0 | 45 | 81 | 100 | 100 | 99 | 98 |
| Example 17 | 0 | 17 | 34 | 94 | 101 | 101 | 99 |
| Example 18 | 0 | 10 | 21 | 54 | 85 | 97 | 97 |

### Experimental Example 6 (dissolution test)

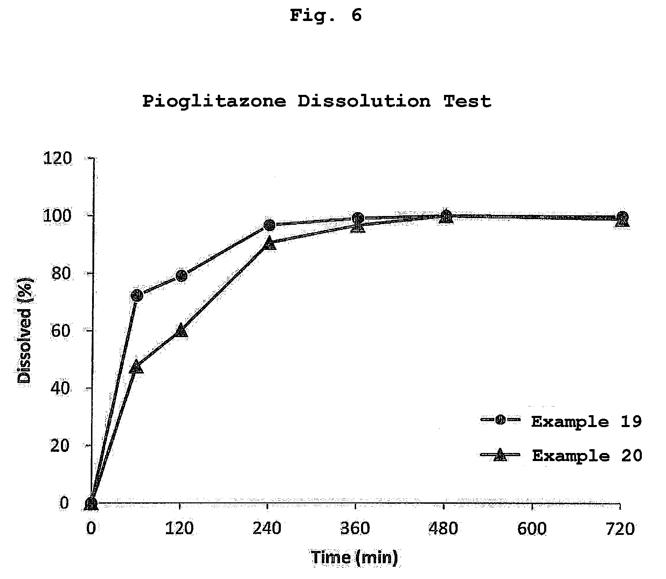

The tablets obtained in Examples 19 - 20 were measured for dissolution property. One tablet was dropped in 900 mL of 0.3M potassium chloride buffer (pH 2), and evaluated by the USP paddle method at rotation number of 50 rpm, 37°C. After dropping the tablet, the test solution was sampled over time, filtered through a non-aqueous filter (0.45 µm), and quantified by the high-speed liquid column chromatography (HPLC) method under the following conditions, and the dissolution rate was calculated. The results are shown in Fig. 6 and Table 13.

### <HPLC conditions>

detector: UV absorption spectrophotometer, measurement
wavelength: 269 nm
column: YMC-Pack ODS-A A-302, 5 µm, inner diameter: 4.6 mm,
length: 75 mm
column temperature: 25°C
mobile phase: acetonitrile:0.1 mol/L ammonium acetate
buffer:glacial acetic acid mixed solution (25:25:1)
flow: 0.7 mL/min

**[Table 13]**

| Time (min) | 0 | 60 | 120 | 240 | 360 | 480 | 720 |
|---|---|---|---|---|---|---|---|
| Example 19 | 0 | 72 | 79 | 97 | 99 | 100 | 100 |
| Example 20 | 0 | 48 | 60 | 91 | 97 | 100 | 99 |

### Experimental Example 7 (dog pharmacokinetic test)

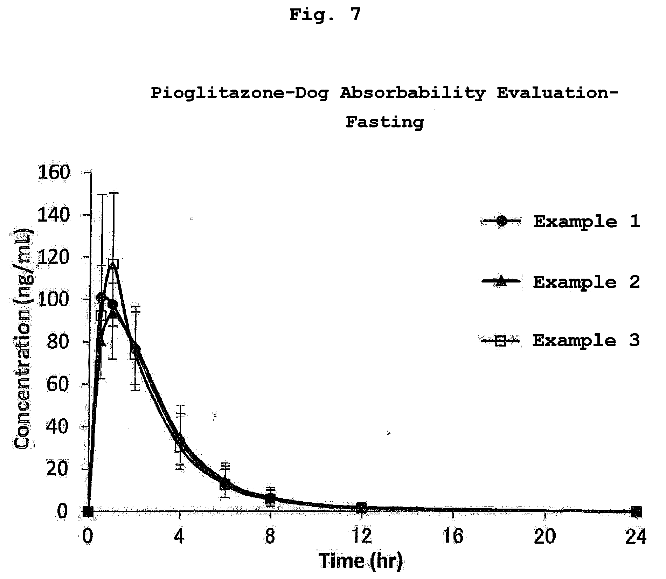

The tablets obtained in Examples 1 - 3 were orally administered to beagles under fasting conditions, and the kinetics in blood was measured. The plasma concentration before administration and 0.5, 1, 2, 4, 6, 8, 12 hr and 24 hr after administration was measured, and the area under the blood drug concentration-time curve (AUC) was calculated by the trapezoidal rule. The results are shown in Fig. 7 and Table 14.

**[Table 14]**

| | **Dose (mg/head)** | **Tₘₐₓ (hr)** | **Cₘₐₓ (ng/mL)** | **AUC₀₋₂₄ₕᵣ (ng·min/mL)** |
|---|---|---|---|---|
| **Example 1** | 1 | 0.7 | 105.8 | 365.1 |
| **Example 2** | 1 | 1.2 | 96.2 | 359.9 |
| **Example 3** | 1 | 0.8 | 120.4 | 359.5 |

### Experimental Example 8 (dog pharmacokinetic test)

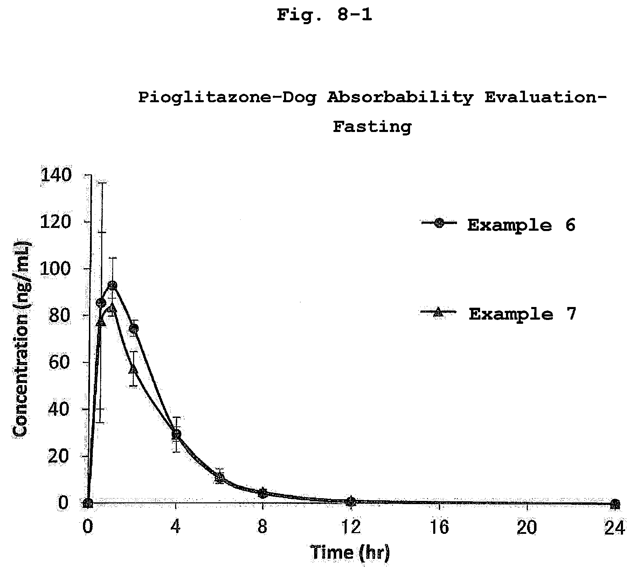

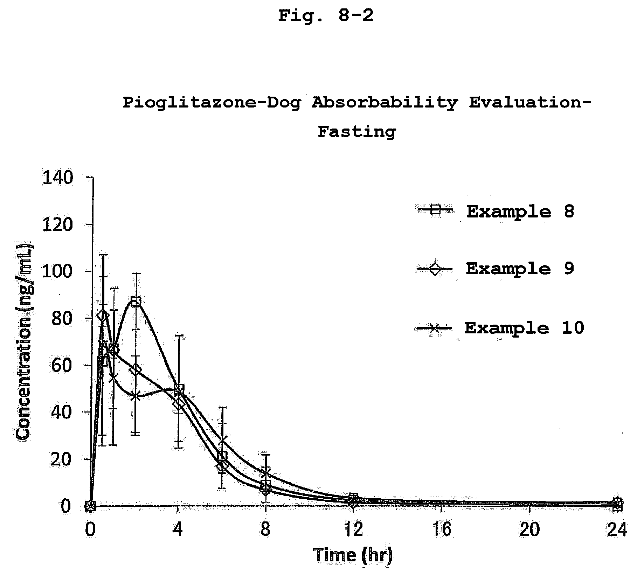

The capsules obtained in Examples 6 - 10 were orally administered to beagles under fasting conditions, and the kinetics in blood was measured. The plasma concentration before administration and 0.5, 1, 2, 4, 6, 8, 12 hr and 24 hr after administration was measured, and the area under the blood drug concentration-time curve (AUC) was calculated by the trapezoidal rule. The results are shown in Figs. 8-1, 8-2 and Table 15.

**[Table 15]**

| | **Dose (mg/head)** | **Tₘₐₓ (hr)** | **Cₘₐₓ (ng/mL)** | **AUC₀₋₂₄ₕᵣ (ng·min/mL)** |
|---|---|---|---|---|
| **Example 6** | 1 | 0.6 | 103.6 | 324.0 |
| **Example 7** | 1 | 0.8 | 94.7 | 293.7 |
| **Example 8** | 1 | 1.4 | 88.0 | 401.1 |
| **Example 9** | 1 | 0.8 | 84.1 | 341.2 |
| **Example 10** | 1 | 1.2 | 85.8 | 374.7 |

### Experimental Example 9 (dog pharmacokinetic test)

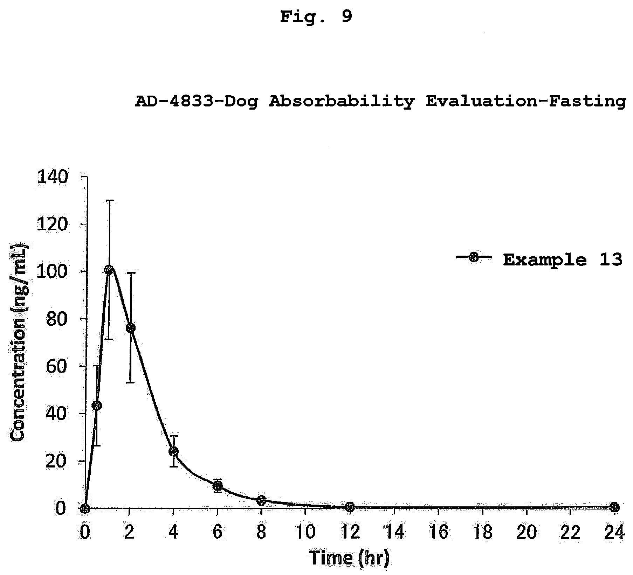

The capsule obtained in Example 13 was orally administered to beagles under fasting conditions, and the kinetics in blood was measured. The plasma concentration before administration and 0.5, 1, 2, 4, 6, 8, 12 hr and 24 hr after administration was measured, and the area under the blood drug concentration-time curve (AUC) was calculated by the trapezoidal rule. The results are shown in Fig. 9 and Table 16.

**[Table 16]**

| | **Dose (mg/head)** | **Tₘₐₓ (hr)** | **Cₘₐₓ (ng/mL)** | **AUC₀₋₂₄ₕᵣ (ng·min/mL)** |
|---|---|---|---|---|
| **Example 13** | 1 | 1.0 | 100.7 | 298.0 |

### Experimental Example 10 (dog pharmacokinetic test)

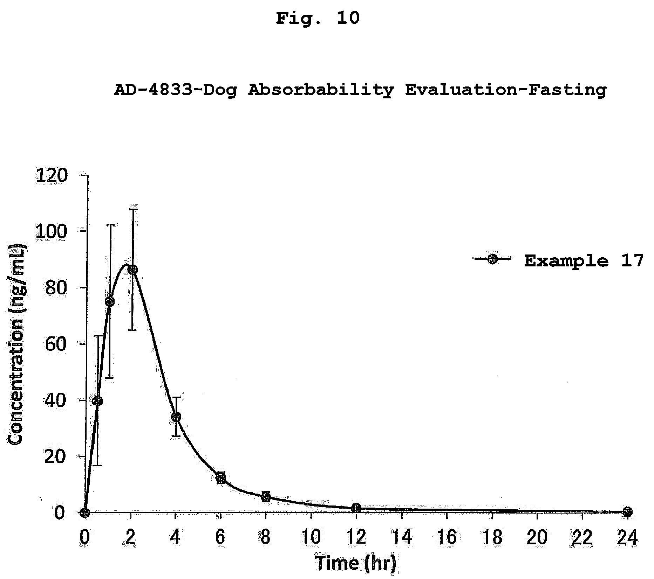

The capsule obtained in Example 17 was orally administered to beagles under fasting conditions, and the kinetics in blood was measured. The plasma concentration before administration and 0.5, 1, 2, 4, 6, 8, 12 hr and 24 hr after administration was measured, and the area under the blood drug concentration-time curve (AUC) was calculated by the trapezoidal rule. The results are shown in Fig. 10 and Table 17.

**[Table 17]**

| | **Dose (mg/head)** | **Tₘₐₓ (hr)** | **Cₘₐₓ (ng/mL)** | **AUC₀₋₂₄ₕᵣ (ng·min/mL)** |
|---|---|---|---|---|
| **Example 17** | 1 | 1.4 | 93.6 | 332.1 |

### Industrial Applicability

The controlled-release solid preparation containing pioglitazone or a salt thereof of the present invention has the following characteristics and is useful in the medical field. (1) It is a preparation capable of sustained release of a drug, which is expected to afford stable efficacy by a sustained release of the drug even when the dose thereof is low. (2) It can control Cmax (e.g., can suppress to a lower level than immediate-release preparation). (3) It can achieve AUC comparable to that of an immediate-release preparation. (4) It is also expected to provide a preparation capable of standing physical stimulation by diet (not easily influenced by diet).

This application is based on patent application No. 2013-088940 filed in Japan, the contents of which are encompassed in full herein.

## Claims

1. A controlled-release solid preparation comprising pioglitazone or a salt thereof in an amount of 0.1 - 8 mg as pioglitazone per preparation unit, which is composed of an immediate-release part, and a sustained-release part in combination.

2. The controlled-release solid preparation according to claim 1, wherein pioglitazone or a salt thereof is contained in one or each of the immediate-release part and the sustained-release part.

3. The controlled-release solid preparation according to claim 2, having a structure wherein the immediate-release part and the sustained-release part are laminated.

4. The controlled-release solid preparation according to claim 3, wherein pioglitazone or a salt thereof is contained in each of the immediate-release part and the sustained-release part.

5. The controlled-release solid preparation according to claim 4, wherein a weight ratio of pioglitazone or a salt thereof contained in the immediate-release part and pioglitazone or a salt thereof contained in the sustained-release part is 1:0.1 - 1:10 in the amount of pioglitazone.

6. The controlled-release solid preparation according to claim 4, wherein a weight ratio of pioglitazone or a salt thereof contained in the immediate-release part and pioglitazone or a salt thereof contained in the sustained-release part is 1:0.5 - 1:2 in the amount of pioglitazone.

7. The controlled-release solid preparation according to any of claims 3 to 6, comprising a gel forming polymer in the sustained-release part.

8. The controlled-release solid preparation according to any of claims 4 to 7, having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.

9. The controlled-release solid preparation according to claim 2, having a structure wherein one of the immediate-release part and the sustained-release part constitutes an inner core and the other constitutes an outer layer covering the inner core.

10. The controlled-release solid preparation according to claim 9, wherein the inner core has a tablet structure.

11. The controlled-release solid preparation according to claim 10, wherein pioglitazone or a salt thereof is contained in each of the immediate-release part and the sustained-release part.

12. The controlled-release solid preparation according to claim 11, wherein a weight ratio of pioglitazone or a salt thereof contained in the immediate-release part and pioglitazone or a salt thereof contained in the sustained-release part is 1:0.1 - 1:10 in the amount of pioglitazone.

13. The controlled-release solid preparation according to claim 11, wherein a weight ratio of pioglitazone or a salt thereof contained in the immediate-release part and pioglitazone or a salt thereof contained in the sustained-release part is 1:0.5 - 1:2 in the amount of pioglitazone.

14. The controlled-release solid preparation according to any of claims 10 to 13, comprising a gel forming polymer in the sustained-release part.

15. The controlled-release solid preparation according to any of claims 10 to 14, having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.

16. The controlled-release solid preparation according to claim 9, wherein the inner core has a tablet structure and the outer layer has a membrane structure.

17. The controlled-release solid preparation according to claim 16, wherein the inner core is the immediate-release part and the outer layer is a sustained-release coating layer comprising a water-soluble polymer and an insoluble polymer.

18. The controlled-release solid preparation according to claim 17, wherein pioglitazone or a salt thereof is contained only in the immediate-release part.

19. The controlled-release solid preparation according to any of claims 16 to 18, having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at 37°C, with rotation number of 50 rpm using a 0.3M potassium chloride buffer (pH 2) as a test solution.

20. The controlled-release solid preparation according to any of claims 3 to 19, which is a tablet.

21. The controlled-release solid preparation according to claim 2, wherein one or each of the immediate-release part and the sustained-release part has a granule structure.

22. The controlled-release solid preparation according to claim 21, wherein the each of the immediate-release part and the sustained-release part has a granule structure.

23. The controlled-release solid preparation according to claim 22, wherein the sustained-release part is a sustained-release granule obtained by coating an immediate-release granule with a coating layer containing a polymer providing sustained release property.

24. The controlled-release solid preparation according to claim 22, wherein the sustained-release part is a sustained-release granule obtained by coating an immediate-release granule with a coating layer containing an enteric polymer.

25. The controlled-release solid preparation according to claim 24, wherein the coating layer containing an enteric polymer is dissolved at pH 5 - 7.

26. The controlled-release solid preparation according to claim 24, wherein the coating layer containing an enteric polymer is dissolved at pH 5.5 - 6.5.

27. The controlled-release solid preparation according to any of claims 22 to 26, wherein pioglitazone or a salt thereof is contained in each of the immediate-release part and the sustained-release part.

28. The controlled-release solid preparation according to claim 27, wherein a weight ratio of pioglitazone or a salt thereof contained in the immediate-release part and pioglitazone or a salt thereof contained in the sustained-release part is 1:0.1 - 1:3 in the amount of pioglitazone.

29. The controlled-release solid preparation according to claim 27, wherein a weight ratio of pioglitazone or a salt thereof contained in the immediate-release part and pioglitazone or a salt thereof contained in the sustained-release part is 1:0.5 - 1:1 in the amount of pioglitazone.

30. The controlled-release solid preparation according to any of claims 22 and 24 to 29, having a dissolution rate of pioglitazone of 10-90% on average at 2 hr time point and 30-110% on average at 4 hr time point, preferably 20-80% on average at 2 hr time point and 40-110% on average at 4 hr time point, in a dissolution test comprising dropping one capsule obtained by filling granules constituting the immediate-release part and granules constituting the sustained-release part in 900 mL of a hydrochloric acid solution (pH 1.2), evaluating same by the USP basket method at rotation number of 100 rpm, 37°C and, 2 hr after dropping the capsule, changing the test solution to 900 mL of a phosphate buffer (pH 6.8)/0.1% CTAB solution and evaluating same under similar conditions.

31. The controlled-release solid preparation according to claim 21, having a granule structure wherein the immediate-release part has a granule structure, and the sustained-release part constitutes an outer layer covering the granule structure.

32. The controlled-release solid preparation according to claim 31, having a granule structure wherein the immediate-release part has a granule structure, and the sustained-release part is applied thereon as a coating layer.

33. The controlled-release solid preparation according to claim 32, wherein the sustained-release part is a coating layer containing a polymer providing sustained release property.

34. The controlled-release solid preparation according to claim 33, wherein the polymer providing sustained release property is a combination of a water-soluble polymer and an insoluble polymer.

35. The controlled-release solid preparation according to claim 32, wherein the sustained-release part is a coating layer containing an enteric polymer.

36. The controlled-release solid preparation according to any of claims 31 to 35, wherein pioglitazone or a salt thereof is contained only in the immediate-release part.

37. The controlled-release solid preparation according to any of claims 31 to 34 and 36, having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP basket method at rotation number of 100 rpm, 37°C using a 0.3M potassium chloride buffer (pH 2) as a test solution.

38. The controlled-release solid preparation according to any of claims 21 to 37, which is a capsule filled with the immediate-release part and the sustained-release part.

39. The controlled-release solid preparation according to claim 21, which is a tablet comprising the immediate-release part and the sustained-release part dispersed therein.

40. The controlled-release solid preparation according to claim 21, which has a structure wherein the sustained-release part has a granule structure, the immediate-release part constitutes an outer layer covering same, and the sustained-release part is dispersed in the outer layer.

41. A controlled-release solid preparation, having a structure wherein an inner core has a layer structure wherein a highly swellable polymer layer and one or plural immediate-release layers containing pioglitazone or a salt thereof in an amount of 0.1 - 8 mg as pioglitazone per preparation unit are laminated, the inner core is coated with a semipermeable membrane as an outer layer, and the semipermeable membrane on the drug-containing immediate-release layer side of the inner core has a minute hole for drug release.

42. The controlled-release solid preparation according to claim 41, which is a tablet.

43. A controlled-release solid preparation comprising a water-swellable gel forming polymer, a water-expandable foaming agent component, and pioglitazone or a salt thereof in an amount of 0.1 - 8 mg as pioglitazone per preparation unit.

44. The controlled-release solid preparation according to claim 43, which has a matrix structure wherein the water-swellable gel forming polymer, the water-expandable foaming agent component, and pioglitazone or a salt thereof are dispersed.

45. The controlled-release solid preparation according to claim 43, comprising a volume swelling preparation part (A) containing the water-expandable foaming agent component, and a preparation part (B) containing the water-swellable gel forming polymer and pioglitazone or a salt thereof, which is laminated on the preparation part (A) or embedded in the preparation part (A) such that a part thereof is exposed.

46. The controlled-release solid preparation according to any of claims 43 to 45, having a dissolution rate of pioglitazone of 20-100% on average at 2 hr time point and 50-110% on average at 4 hr time point, preferably 25-60% on average at 2 hr time point and 60-100% on average at 4 hr time point, and 80-110% on average at 6 hr time point, in a dissolution test according to the USP paddle method at rotation number of 50 rpm, 37°C using a 0.3M potassium chloride buffer (pH 2) as a test solution.

47. The controlled-release solid preparation according to any of claims 1 to 46, comprising pioglitazone or a salt thereof in an amount of 0.5 - 8 mg as pioglitazone per preparation unit.

48. The controlled-release solid preparation according to any of claims 1 to 46, comprising pioglitazone or a salt thereof in an amount of 0.5 - 6 mg as pioglitazone per preparation unit.

49. The controlled-release solid preparation according to any of claims 1 to 46, comprising pioglitazone or a salt thereof in an amount of 0.5 - 1 mg as pioglitazone per preparation unit.

50. The controlled-release solid preparation according to any of claims 1 to 49, which has Cmax of 20 - 140 ng/mL and the area under the blood drug concentration-time curve (AUC₀₋₂₄ₕᵣ) of 100 - 510 ng/mL on administration of 1 mg of pioglitazone or 1 mg based on pioglitazone, when pioglitazone is orally administered to a pentagastrin-treated beagle under fasting conditions and kinetics of pioglitazone in blood is measured after the administration, and AUC₀₋₂₄ₕᵣ is calculated by the trapezoidal rule based on the measurement results of plasma concentration before administration, and after 0.5, 1, 2, 4, 6, 8, 12 hr and 24 hr.

51. A method for the prophylaxis and/or treatment of Alzheimer's dementia, comprising administering the controlled-release solid preparation according to any of claims 1 to 50 to a subject in need of the administration.
